(19) Europäisches Patentamt / European Patent Office / Office européen des brevets

(11) **EP 3 003 281 B1**

(12) **EUROPEAN PATENT SPECIFICATION**

(45) Date of publication and mention
of the grant of the patent:
**19.09.2018 Bulletin 2018/38**

(21) Application number: **14807663.1**

(22) Date of filing: **04.06.2014**

(51) Int Cl.:
*A61K 9/19* (2006.01)     *A61K 38/16* (2006.01)
*A61K 47/06* (2006.01)     *A61P 31/12* (2006.01)
*A61P 35/02* (2006.01)     *B82Y 5/00* (2011.01)
*A61K 47/42* (2017.01)     *A61K 9/00* (2006.01)
*A61K 9/16* (2006.01)     *A61K 9/51* (2006.01)
*A61K 31/203* (2006.01)     *A61K 31/4725* (2006.01)

(86) International application number:
**PCT/US2014/040966**

(87) International publication number:
**WO 2014/197640 (11.12.2014 Gazette 2014/50)**

(54) **NOVEL CORE-SHELL NANOPARTICLES FOR ORAL DRUG DELIVERY**

NEUARTIGE KERN-HÜLLE-NANOPARTIKEL ZUR ORALEN WIRKSTOFFVERABREICHUNG

NOUVELLES NANOPARTICULES COEUR-ÉCORCE POUR ADMINISTRATION ORALE DE MÉDICAMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.06.2013 US 201361830947 P
22.01.2014 US 201461930154 P**

(43) Date of publication of application:
**13.04.2016 Bulletin 2016/15**

(73) Proprietor: **South Dakota State University
Brookings, SD 57006 (US)**

(72) Inventors:
• **PERUMAL, Omathanu
Brookings, SD 57006 (US)**
• **ALQAHTANI, Mohammed, Saeed A.
Riyadh 11451 (SA)**

(74) Representative: **advotec.
Patent- und Rechtsanwälte
Bahnhofstrasse 5
94315 Straubing (DE)**

(56) References cited:
WO-A2-2012/116272     US-A1- 2012 195 947
US-A1- 2012 315 306

• ASHOK R. PATEL ET AL: "Sodium Caseinate Stabilized Zein Colloidal Particles", JOURNAL OF AGRICULTURAL AND FOOD CHEMISTRY, vol. 58, no. 23, 8 December 2010 (2010-12-08), pages 12497-12503, XP55355071, US ISSN: 0021-8561, DOI: 10.1021/jf102959b
• HUAIQIONG CHEN ET AL: "Processes improving the dispersibility of spray-dried zein nanoparticles using sodium caseinate", FOOD HYDROCOLLOIDS, vol. 35, 1 March 2014 (2014-03-01), pages 358-366, XP55202386, ISSN: 0268-005X, DOI: 10.1016/j.foodhyd.2013.06.012
• KALHAPURE RS ET AL.: 'A novel biocompatible bicephalous dianionic surfactant from oleic acid for solid lipid nanoparticles.' COLLOIDS SURF B BIOINTERFACES vol. 105, 06 January 2013, pages 215 - 222, XP028523535 DOI: 10.1016/J.COLSURFB.2013.01.011
• SHAH LK ET AL.: 'Intracellular delivery of saquinavir in biodegradable polymeric nanoparticles for HIV/AIDS.' PHARM RES. vol. 23, no. 11, 13 September 2006, pages 2638 - 2645, XP019453315 DOI: 10.1007/S11095-006-9101-7

**Description**

FIELD OF THE INVENTION

The present invention relates generally to drug delivery technologies, and more specifically to an oral nanoparticle drug delivery system, including methods for preparing such a system using a hydrophobic water insoluble protein, which nanoparticles may include prolamine to generate an oral drug delivery system.

BACKGROUND INFORMATION

Despite estimates of a pharmaceutical market for prescription pediatric drugs of $43 billion every year, the lack of pediatric-friendly formulations results in 40% of the world's population at elevated risk for inappropriate dosing, noncompliance, toxicity and difficulty access to most drugs. For example, all-trans-retinoic acid (ATRA) is used in the treatment of acute promyelocytic leukemia in children, however, current formulations suffer from poor chemical stability, poor water solubility and poor oral bioavailability.

Given that more than 3.3 million HIV patients are children, most of pediatric HIV patients do not have access to proper medication. Pediatric HIV is very difficult to eliminate due to maternal to fetus transmission and breastfeeding mainly in developed countries. The latest World Health Assembly (WHA) has recognized the right of pediatric patients by promoting the need for safe and effective medicines under the global umbrella 'Make medicines child size'.

Saquinavir, is a highly lipophilic protease inhibitor used in the treatment of HIV and classified in class IV of the Biopharmaceutic Classification System (BCS). However, saquinavir suffers from problems of bitter taste to poor solubility, and permeability leading to low bioavailability of approximately (0.7- 4%).

Zein, a plant protein that can be isolated from corn or maize, belongs to a family of prolamines that are composed of high amounts of non-polar amino acids, such as proline, glutamine and asparagine. Zein is odorless, non-toxic, biodegradable and water-insoluble, and is therefore an attractive component for many applications, including use in the pharmaceutical and medical industries. In the pharmaceutical industries, zein has been used, for example, to film-coat materials and to form particulate systems such as microparticles or nanoparticles (U.S Patent No. 5,679,377 (Bernstein et al.), Liu et al., Biomaterials 26 (2005) 109-115; Lopez and Murdan, J Microencapsulation 23 (2006) 303-314; Zhong et al., Food Biophysics 3 (2008) 186-190; Parris et al., J Agric Food Chemistry 53 (2005) 4788-4792).

US 2012/195947 A1 describes retinol-loaded zein nanoparticles stabilized with β-casein as well as a production of such nanoparticles with phase separation or lyophilization.

Ashok R. Patel et al.: "Sodium Caseinate Stabilized Zein Colloidal Particles", Journal of Agricultural and Food Chemistry, vol. 58, no. 23, 8 December 2010 (2010-12-08), pages 12497-12503, XP55355071, US ISSN: 0021-8561, DOI: 10.1021/jf102959b discloses nanoparticles comprising zein and casein as pharmaceutical delivery systems.

Huaiqiong Chen et al.: "Processes improving the dispersibility of spray-dried zein nanoparticles using sodium caseinate", Food Hydrocolloids, vol. 35, 1 March 2014 (2014-03-01), pages 358-366, XP55202386, ISSN: 0268-005X, DOI: 10.1016/j.foodhyd.2013.06.012 describes processes for improving the dispersibility of spray-dried zein nanoparticles using sodium caseinate.

Particulate drug delivery systems can be used for taste masking, improving drug stability and drug solubility. The ease of medication administration, safety and palatability of formulation excipients are the most important determinants for dosage forms, especially for pediatric use.

In general, for pediatric drugs, challenges remain with respect to the safety and palatability of excipients used in pediatric formulations, ease of administration and patient compliance, special storage conditions, compatibility with foods and beverages, and engineering pediatric drug formulations with consideration of pediatric GI physiology.

Accordingly, new methods are needed for preparing zein particles to render the particles useful for oral administration. Also needed are new therapeutic carriers for the delivery of important therapeutics in a safe and effective manner, so as to overcome challenges associated with pediatric oral administration.

SUMMARY OF THE INVENTION

Applicants have developed an oral nanoparticle drug delivery system for pediatric patients using food-grade polymers. To this end nanoparticles were developed using, for example, zein, a corn protein as the hydrophobic core and casein, a milk protein or a globular glycoprotein, such as lactoferrin, as the shell.

In embodiments, a nanoparticle is disclosed including at least two proteins, where a first protein is a prolamine and a second protein is lactoferrin, and where the nanoparticle exhibits a core-shell structure.

In one aspect, the prolamine protein comprises white zein, yellow zein, gliadin, hordein, or kafirin.

In another aspect, the nanoparticle may further include a cargo molecule, where the cargo molecule is a class II or class IV drug. In a related aspect, the cargo molecule includes a pharmaceutical material, a therapeutic material, a diagnostic agent, agricultural material, an immuno-potentiating agent, a bioactive agent, and combinations thereof.

In one aspect, the cargo molecule includes retinol, 13-trans-retinoic acid (tretinoin) or all trans retinoic acid (ATRA), 13-cis-retinoic acid (isotretinoin), 9-cis-retinoic acid (alitretinoin), retinaldehyde, etretnate, acitretin, α-carotene, β-carotene, γ-carotene, β-cryptozanthin, lutein, zeaxanthin, and combinations thereof.

In another aspect, the cargo molecule is saquinavir.

In one aspect, the nanoparticle is formed by spray drying or phase separation.

In another aspect, the nanoparticle further includes cargo, where the cargo is a cell, protein, nucleic acid, antibody, growth factor, or a combination thereof. In a related aspect, the cargo is adsorbed to the surface of the nanoparticle.

In one aspect, the nanoparticle is cross-linked, where a crosslinking agent includes genipin.

In another aspect, the prolamine protein of the nanoparticle is PEGylated.

In one aspect, the nanoparticle is in the form of a dry, free flowing, colorless or white, non-hygroscopic powder. In another aspect, the nanoparticle includes a diluent, an excipient, or carrier to form a pharmaceutically acceptable composition. In a related aspect, the composition is an oral formulation and is optionally contained in a food or a beverage.

In one embodiment, a kit is disclosed including a lyophilized powder or dispersion containing the nanoparticles as disclosed herein; one or more buffers; one or more labels; one or more containers; and an instruction manual, where the instruction manual discloses how to use the lyophilized powder.

Further, a method of preparing a nanoparticle is disclosed including dissolving a prolamine protein in a hydroalcoholic solvent to form an organic phase; adding the organic phase to a buffer, where the buffer includes a citrate anion, a separate protein, optionally at least one cargo molecule, or optionally a stabilizing molecule including a gum, a polysaccharide or a pectin, or a combination thereof, to form a precipitate; sonicating the precipitate; centrifuging the remaining aqueous phase to form a pellet; washing the pellet, optionally adding a cryoprotectant; and lyophilizing the pellet, where the resulting nanoparticle has a particle size of between about 90 nm to about 300 nm.

In one aspect, the separate protein is lactoferrin and the polysaccharide is dextran or gum arabic.

In one embodiment, a method of preparing a nanoparticle is disclosed including dissolving a prolamine protein and a second protein in a hydroalcoholic solvent including a buffer to form a precipitate, where the buffer includes a citrate anion; sonicating the precipitate to form a sonicate; optionally adding one or more cargo molecules to the sonicate to form a mixture; loading the sonicate or mixture into a spray drier, spray drying the sonicate or mixture into a collecting drum to form a spray dried material; and collecting the spray dried material from the collecting drum, where the resulting nanoparticle has a particle size of between less than about 90 nm to about 300 nm.

In a related aspect, where the separate protein is lactoferrin.

In one embodiment, the use of the nanoparticle as described is disclosed for the preparation a medicament for the treatment of a disorder in a subject in need thereof, where the disorder includes acute myeloid leukemia, promyelocytic leukemia, neuroblastoma, and pediatric HIV. The scope of the invention is defined by the claims. Any references in the description to methods of treatment refer to the compounds, pharmaceutical compositions and medicaments of the present invention for use in a method for treatment of the human (or animal) body by therapy (or for diagnosis).

In another embodiment, a method of treating a disorder is disclosed including orally administering the nanoparticle as described herein to a subject in need thereof, where the disorder includes acute myeloid leukemia, promyelocytic leukemia, neuroblastoma, and pediatric HIV.

## BRIEF DESCRIPTION OF THE DRAWINGS

The following drawings form part of the specification and are included to further demonstrate certain embodiments or various aspects of the invention. In some instances, embodiments of the invention can be best understood by referring to the accompanying drawings in combination with the detailed description presented herein. The description and accompanying drawings may highlight a certain specific example, or a certain aspect of the invention, however, one skilled in the art will understand that portions of the example or aspect may be used in combination with other examples or aspects of the invention.

FIG. 1. illustrates steps for the preparation of zein nanoparticles stabilized by lactoferrin.

FIG. 2 illustrates by means of a flow chart general steps for preparing genipin cross-linked zein-casein nanoparticles, not according to the invention.

FIG. 3 illustrates a flow chart for spray drying.

FIG. 4 shows a graph of in vitro release date of [3H]-ATRA from ZLF nanopartilces.

FIG. 5 shows a graph of SQ release in food matrices followed by SGF and SIF. (A) milk pre-administration and (B) apple juice pre-administration.

FIG. 6 shows a graph illustrating Papp values over time for SQ (saquinavir), ZC (zein-casein), ZLF (zein-lactoferrin), and PZ (polyethylene glycol).

FIG. 7 shows a graph illustrating Papp values over time for ZC (zein-casein), ZLF (zein-lactoferrin), and PZ (polyethylene glycol).

FIG. 8 shows the effect of nanoparticles on TEER.

FIG. 9 shows graphs for cellular uptake as a function of time (A) and temperature (B).

FIG. 10 shows data from a competitive inhibition assay.

FIG. 11 shows P-gp inhibition in CaCo-2 cells.

FIG. 12 shows plasma concentration profile of saquinavir in rat after oral administration of saquinavir loaded in zein-casein (ZC) nanoparticles; zein lactoferin (ZLF) nanoparticles and PEG-zein (PZ) nanoparticles. The bioavailability of saquinavir was enhanced from zein based nanocarriers. As can be seen from the profile, the drug was slowly release and the plasma concentration was maintained for 48hrs in the plasma.

## DETAILED DESCRIPTION OF THE INVENTION

The oral route is the most prevalent means of delivering medications to the pediatric population. However, the lack of child-friendly formulations results in 40% of the world's population at elevated risk of inappropriate dosing and difficulty in accessing most APIs (Active Pharmaceutical Ingredient). To this end, the natural food-grade polymers (e.g., proteins) are promising drug delivery carriers. Of the biopolymers, zein, a globular hydrophobic protein from corn, and β-casein, a linear amphipathic protein from milk, are widely used as ingredients in food and pharmaceutical applications. These polymers are biodegradable, widely available and approved by US-FDA, as a GRAS material. In embodiments, core-shell nanoparticles using zein and casein for pediatric oral drug delivery applications are disclosed.

In embodiments, the protein based nanoparticles of the present disclosure can efficiently load BCS class II/IV drugs and achieve successful oral delivery of these drugs to subjects in need thereof. As is well known in the art, BCS class II drugs are those having high permeability, low solubility; BCS class IV drugs are those having low permeability and low solubility.

BCS class II drugs include, but are not limited to, amiodarone, atorvastatin, azithromycin, carbamazepine, carvedilol, chlorpromazine, cisapride, ciprofloxacin, cyclosporine, danazol, dapsone, diclofenac, diflunisal, digoxin, erythromycin, flurbiprofen, glipizide, glyburide, griseofulvin, ibuprofen, indinavir, indomethacin, itraconazole, ketoconazole, lansoprazole I, lovastatin, mebendazole, naproxen, nelfinavir, ofloxacin, oxaprozin, phenazopyridine, phenytoin, piroxicam, raloxifene, ritonavir, saquinavir, sirolimus, spironolactone, tacrolimus, talinolol, tamoxifen, and terfenadine.

BCS class IV drugs include, but are not limited to, amphotericin, chlorthalidone, chlorothiazide, colistin, ciprofloxacin, furosemide, hydrochlorothiazide, mebendazole, methotrexate, neomycin.

In embodiments, all-trans retionic acid (ATRA), a drug used for neuroblastoma in pediatrics may be combined with said nanoparticles, which combination overcomes problems associated with poor chemical stability, solubility and oral bioavailability.

In embodiments, the protein based nanoparticles of the present disclosure have a core-shell structure. In one aspect, said protein based nanoparticles may be formed by phase separation. In a related aspect, the core-shell particles are formed due to differences in hydrophobicity between the proteins comprising the nanoparticles.

In embodiments, the protein based nanoparticles of the present disclosure may be made by spray drying. In a related aspect, the spray drier that may be used is a Nano Spray Dryer B-90, BÜCHI (BUCHI Labortechnik AG, Switzerland).

In embodiments, the spray rate may be 100%, about 50%, or about 20%. In a related aspect, the nitrogen flow rate may be about 90L/min, abou120 L/min or about 150L/min. In another aspect, the inlet temperature may be about 70°C, about 90°C or about 120°C.

In embodiments, the spray drying process comprise an inlet temperature of about 155°C, a nitrogen flow rate of about 90L/min, and spray mesh size of about 4 μm and a spray rate of about 100% Nanoparticles are disclosed herein using zein, a corn protein as the hydrophobic core and casein, a milk protein as the shell.

In another aspect, the protein component comprises a prolamine selected from the group consisting of zein, gliadin, hordein, or kafirin. In a related aspect, the prolamine is zein. In another related aspect, the zein protein is combined with lactoferrin or polyethylene glycol (PEG), where the PEG is from about 3 to about 20 kDa.

In embodiments, the zein/lactoferrin is present at a ration of about 1:1 or about 1:2 or about 1:3 or about 1:4 or about 1:5. In one aspect, the nanoparticles are made in a solution comprising a citrate buffer, having a pH of about 6.8 to about 7.4. In a related aspect the solution may comprise a hydroalcoholic phase, where the alcoholic phase is between about 70% to about 90% alcohol.

In embodiments, the particles have a size in the nanometer range, with a narrow polydispersity. In one aspect, the average size of said nanoparticles is between about 50 to about 70 nm, about 70 to about 90 nm, about 90 to about 115 nm, about 115 to about 150 nm, about 150 to about 200 nm, about 200 to about 300 nm, about 300 to about 350 nm, about 300 to about 500 nm, or about 500 to about 900 nm.

In another aspect, the nanoparticles exhibit an encapsulation efficiency of about 71.06% to about 90%, and a loading efficiency of about 1.17% to about 9%. In a related aspect, the particles exhibit a smooth and spherical surface.

In another aspect, the protein based nanoparticles comprises stabilizers, which stabilizers include, but are not limited to, PEG, gums and pectins, where such stabilizers are combined with the protein in spray drying or phase separation.

In embodiments, the processes as disclosed herein may be optimized to achieve the desired particle size, PDI and high encapsulation efficiency. In a related aspect, optimization may involve the use of $3^3$ Box-Behken design. In a related aspect, optimized nanoparticle design using a model comprising quadratic equations, resulted in a formulation that was optimized on the basis of observed and predicted values.

In embodiments, the nanoparticle formulations may be used as an oral controlled drug delivery system for ATRA. In a related aspect, the stability of ATRA is increased in ATRA loaded nanoparticles of the present disclosure. In another aspect, uptake of the nanoparticles may be carried out with cells in vitro, including but not limited to Caco-2 polarized cells. Further, results from such studies may be confirmed using in-situ perfusion analysis and segmental intestinal uptake in suitable animal models, including rats. Moreover, pharmacokinetic analysis may be carried out using said animals.

In embodiments, the nanoparticle formulations may be used as an oral controlled drug delivery system for saquinavir.

Applicants have successfully addressed the oral delivery issues of retinol by encapsulating retinol in novel protein based nanocarriers for oral dosage applications, including pediatric use.

A unique aspect of nanocarriers is the ability of the nanoparticles to address multiple market challenges for oral delivery of retinol, including providing ease of administration and patient compliance, avoiding special storage conditions, providing compatibility with foods and beverages, and providing pediatric drug formulations with consideration of pediatric GI physiology.

Retinol water dispersibility is significantly increased after encapsulation in nanoparticles. The retinol release can be sustained from zein-β-casein nanoparticles (not according to the invention) leading to lower dose and reduced frequency of application. The encapsulation of retinol in zein-β-casein nanoparticles significantly increases the shelf-life of retinol formulations. Zein-β-casein nanoparticles increase the flowability and dispersibility of retinol in solid and semi-solid formulations. Because retinol is a hygroscopic sticky powder, the encapsulation of retinol in nanoparticles can overcome the difficult handling and processing issues associated with retinol.

Oral drug administration is the most favorable route to deliver medications most importantly to pediatrics. Given that more than 3.3 million HIV patients are children, most of pediatric HIV patients do not have access to proper medication. 1 Pediatric HIV is very difficult to eliminate due to maternal to fetus transmission and breastfeeding mainly in developed countries. The latest World Health Assembly (WHA) has recognized the right of pediatric patients by promoting the need for safe and effective medicines under the global umbrella 'Make medicines child size'. Saquinavir, is a highly lipophilic protease inhibitor used in the treatment of HIV and classified in class II of the Biopharmaceutic Classification System (BCS). However, saquinavir suffers from problems of bitter taste to poor solubility, and permeability leading to low bioavailability of approximately (0.7- 4%). Recent advances in nanotechnology have resulted in the development of nanoparticle based oral formulation for diagnostic and therapeutic purposes.

Lactoferrin (Lf), a mammalian cationic iron-binding glycoprotein belonging to the transferrin (Tf) family, has been widely used in a variety of fields ranging from treating infant diarrhea and supporting newborn growth to food and other applications. It helps in iron transport and increases the iron bioavailability.

With regard to the core-shell architecture of the protein-protein oral nanoparticles, the β casein and lactoferrin are hydrophilic proteins that stabilize the zein core resulting in smaller sized nanoparticles. Further the core shell architecture provides better proteolytic stability as demonstrated by in-vitro stability studies disclosed herein. The cationic charge of lactoferrin helps to further stabilize the zein-lactoferrin nanoparticles. Further, the cationic charge on the surface may be used to adsorb other anonic compounds including drugs, gene or negatively charged proteins.

In case of PEG-zein, nanoparticles prepared by spray drying, different molecular weights of PEG including 3000, 10,000 and 20,000 Da have been shown herein to be useful.

The advantages of nanoparticles formulation over conventional one are notable including increased drug solubility, sustained release, protect the cargo from the harsh acidic or enzymatic environment and specific delivery while minimizing drug toxic effects. Polymers that extensively used for the production of the nanoparticles are Eudragit, gelatin and PLGA.

Proteins used as a platform for nanoparticles have many advantages they are biodegradable, accessible, naturally abundance, chemically modifiable, cost effective, and eco-friendly compared to synthetic excipients currently in the market. on the other hand, cationic polymers, such as chitosan, lysozyme and poly-lysine demonstrated potential mucoadhesive properties as well as cellular internalization efficacy. Such properties were attributed to an electrostatic interaction with the mucus and anionic glycoproteins which were abundant on the surface of GI tract. Zein is a hydrophobic water insoluble protein from corn. It is edibility, taste masking and hydrophobic characteristic makes it a good candidate for oral delivery system. In embodiments, to develop a completely natural and biodegradable delivery system, stabilized zein nanoparticles using milk protein was used. For example, lactoferrin is an iron-binding protein in milk. It has nutritional and biological properties, particularly important for formula-fed infants. In embodiments; nanoparticles with core shell structure are described, including their physicochemical and conformational characteristics. In a related aspect, zein based nanoparticles are disclosed which to enhance the solubility and permeability of BCS class II/IV drugs, including, but not limited to, saquinavir.

Definitions

As used herein, the recited terms have the following meanings. All other terms and phrases used in this specification have their ordinary meanings as one of skill in the art would understand. Such ordinary meanings may be obtained by reference to technical dictionaries, such as Hawley's Condensed Chemical Dictionary 14th Edition, by R.J. Lewis, John Wiley & Sons, New York, N.Y., 2001.

References in the specification to "one embodiment", "an embodiment", etc., indicate that the embodiment described may include a particular aspect, feature, structure, moiety, or characteristic, but not every embodiment necessarily includes that aspect, feature, structure, moiety, or characteristic. Moreover, such phrases may, but do not necessarily, refer to the same embodiment referred to in other portions of the specification. Further, when a particular aspect, feature, structure, moiety, or characteristic is described in connection with an embodiment, it is within the knowledge of one skilled in the art to affect or connect such aspect, feature, structure, moiety, or characteristic with other embodiments, whether or not explicitly described.

The terms "comprising," "including," "having," "containing," "characterized by," and grammatical equivalents thereof, are inclusive or open-ended terms that do not exclude additional, unrecited elements or method steps, but also include the more restrictive terms "consisting of and "consisting essentially of.

The singular forms "a," "an," and "the" include plural reference unless the context clearly dictates otherwise. Thus, for example, a reference to "a compound" (e.g., a drug) includes a plurality of such compounds, so that a compound X includes a plurality of compounds X. As an additional example, reference to "a nanoparticle" can include a plurality of such nanoparticles, and reference to a "molecule" is a reference to a plurality of molecules, and equivalents thereof. It is further noted that the claims may be drafted to exclude any optional element. As such, this statement is intended to serve as antecedent basis for the use of exclusive terminology, such as "solely", "only", in connection with the recitation of claim elements or use of a "negative" limitation.

The term "and/or" means any one of the items, any combination of the items, or all of the items with which this term is associated. The phrase "one or more" is readily understood by one of skill in the art, particularly when read in context of its usage. For example, one or more substituents on a phenyl ring refers to one to five, or one to four, for example if the phenyl ring is disubstituted.

The term "about" or "approximately" means reasonably close to, or a little more or less than, a recited number or amount. Thus, the term "about" can refer to a variation of $\pm$ 5%, $\pm$ 10%, $\pm$ 20%, or $\pm$ 25% of the value specified. For example, "about 50" percent can in some embodiments carry a variation from 45 to 55 percent. For integer ranges, the term "about" can include one or two integers greater than and/or less than a recited integer. Unless otherwise indicated herein, the term "about" is intended to include values, e.g., weight percents, proximate to the recited range that are equivalent in terms of the functionality of the individual ingredient, the composition, or the embodiment. In addition, unless indicated otherwise herein, a recited range (e.g., weight percents or carbon groups) includes each specific value or identity within the range.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range (e.g., weight percents or carbon groups) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

As will also be understood by one skilled in the art, all language such as "up to," "at least," "greater than," "less than," "more than," "or more", include the number recited and such terms refer to ranges that can be subsequently broken down into subranges as discussed above. In the same manner, all ratios recited herein also include all subratios falling within the broader ratio. Accordingly, specific values recited for radicals, substituents, and ranges, are for illustration only; they do not exclude other defined values or other values within defined ranges for radicals and substituents.

One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group. Accordingly, provisos may apply to any of the disclosed categories or embodiments whereby any one or more of the recited elements, species, or embodiments, may be excluded from such categories or embodiments, for example, as used in an explicit negative limitation.

The term "zein" refers to a member of the class of prolamine proteins. Prolamines are found in various grains such as corn, wheat, barley, rice, and sorghum, as well as in other plants and animals. Other examples of prolamines include gliadin, hordein and kafirin. These prolamines can be exchanged for zein in the various embodiments described herein. Zein is composed of a high proportion of non-polar amino acids, such as proline, glutamine and asparagine, and has a molecular weight of about 22-27 kDa (Shukla, Zein: the industrial protein from corn, Ind Crops Prod 13, 171-92 ; 2001), and can be a mixture of three distinct proteins with varying molecular weights. A typical sample of zein can have approximately 20% leucine, 10% proline, 21-26% glutamine, 5% asparagine, and 10% alanine, therefore at least about 61% of its amino acid composition is of hydrophobic amino acids. These hydrophobic amino acids render the protein water insoluble. Zein is a biodegradable US-FDA approved GRAS polymer (Fed Register (1985) 50:8997-8999).

Zein can be manufactured as a powder from corn gluten meal. Pure zein is odorless, tasteless, water-insoluble, and edible, properties which have rendered it an important component for processed foods and pharmaceuticals. Methods for isolating, processing, and using zein are known in the art. See for example, Lawton, Cereal Chem 2002, 79(1): 1-18, and WO2009/137112 (Perumal et al.). A "grade" of zein refers to a variety of types or forms of zein, including white zein and yellow zein, derived by various means, such as is disclosed in U.S. Patent No. 5,254,673 (Cook et al.).

Casein is the name for a family of related phosphoproteins ($\alpha$S1, $\alpha$S2, $\beta$, $\kappa$). These proteins are commonly found in mammalian milk, making up 80% of the proteins in cow milk and between 20% and 45% of the proteins in human milk. Casein has a wide variety of uses, from being a major component of cheese, to use as a food additive, to a binder for safety matches. As a food source, casein supplies amino acids; carbohydrates; and two inorganic elements, calcium and phosphorus.

$\beta$-casein is a major protein found in milk. The protein binds to calcium at its phosphorylated regions, which in turn are highly conserved. The calcium then binds the caseins together and forms micelles, which better enable it to be ingested by infants. It also has opioid type effects on newborn sleeping patterns in humans. The protein, as a whole, is disordered and is characterized as a random coil protein. Beta-casein may be present as one of two major genetic variants: A1 and A2. The major difference between the A1 and A2 beta-casein proteins is a single amino acid at position 67 in a strand of 209 amino acids. A1 beta-casein has the amino acid histidine at position 67, while A2 beta-casein has a proline amino acid in the same position. A1 beta-casein in cow's milk is different to other mammalian beta-caseins, because of its histidine at position 67. Human milk, goat milk, sheep milk and other species' milk contain beta-casein which is 'A2 like', because they have a proline at the equivalent position in their beta-casein chains.

Lactoferrin, also known as lactotransferrin (LTF), is a multifunctional protein of the transferrin family. Lactoferrin is a globular glycoprotein with a molecular mass of about 80 kDa that is widely represented in various secretory fluids, such as milk, saliva, tears, and nasal secretions. Lactoferrin is also present in secondary granules of PMN and is secreted by some acinar cells. Lactoferrin can be purified from milk or produced recombinantly. Human colostrum ("first milk") has the highest concentration, followed by human milk, then cow milk (150 mg/L).

Lactoferrin is one of the components of the immune system of the body; it has antimicrobial activity (bacteriocide, fungicide) and is part of the innate defense, mainly at mucoses. In particular, lactoferrin provides antibacterial activity to human infants.

Lactoferrin interacts with DNA and RNA, polysaccharides and heparin, and shows some of its biological functions in complexes with these ligands.

The term "biocompatible" means that the polymer or conjugate referred to does not cause or elicit significant adverse effects when administered *in vivo* to a subject. Examples of possible adverse effects include excessive inflammation and/or an excessive or adverse immune response, as well as toxicity. For example, zein and beta-casein are biocompatible components.

The term "nanoparticle" is generally known to refer to a particle that is not more than 1000 nm in at least one dimension. However, the nanoparticles formed by the methods described herein will have a diameter of a specified value as defined herein. Further, the use of the term "nanoparticle" is also meant to refer generically to blank nanoparticles and nanoparticles loaded with a molecule and formed by methods of the present invention. As used herein, unless defined otherwise, "blank nanoparticle" refers to nanoparticles that do not have a selected particle, molecule or material formed with or in conjugation with the nanoparticle.

The term "diameter" when used in the context of nanoparticle dimensions refers to the mean linear dimension of the particle for lines passing through the center of mass of the particle. Acceptable approximation of the diameter of non-spherical particles may be provided, for example, by taking the mean of the thickness of the particle along three orthogonal axes of a coordinate system, with one of the axes aligned with the longest dimension of the particle.

The term "hydroalcoholic solvent" refers to a solvent system that includes both water and an alcoholic solvent, such as methanol, ethanol, n-propanol, *iso*-propanol, or butanol (including 1-butanol, 2-butanol (*sec*-butanol), *iso*-butanol, and *tert*-butanol). Common hydroalcoholic solvent systems include 50%, 70%, 90%, and 92% ethanol in water.

The term "contacting" refers to the act of touching, making contact, or of bringing to immediate or close proximity, including at the cellular or molecular level, for example, to bring about a physiological reaction, a chemical reaction, or a physical change, e.g., in a solution, in a reaction mixture, *in vitro* or *in vivo.*

The term "*in vivo*" means of or within the body of a subject, such as that of a patient, and includes administration of nanoparticles by a variety of means including oral, intravenous, intratumorally, peritumorally, intraperitoneal, parenteral, subcutaneous, topical, ocular, pulmonary and nasal routes of administration.

The term "*in vitro*" refers to environments outside of the body of a subject or patient.

The term "*in situ*" refers to the original position; not having been moved or transferred to another location.

The term "associating" refers to the complexing of cargo or cargo molecules to the nanoparticles of the instant disclosure, and include conjugation (covalent or non-covalent) to the surface or interior regions of the particle, adsorption, and encapsulation.

The term "complexing", including grammatical variations thereof, refers to the combination of various cellular or molecular entities with the nanoparticles of the present disclosure.

The term "administered" or "administration", when used in the context of therapeutic and diagnostic uses for nanoparticles, refers to and includes the introduction of a selected amount of nanoparticles into an *in vivo* or *in vitro* environment for the purpose of, for example, delivering a therapeutic agent to a targeted site.

An "effective amount" refers to an amount effective to treat a disease, disorder, and/or condition, or to bring about a recited effect. For example, an amount effective can be an amount effective to reduce the progression or severity of the condition or symptoms being treated. Determination of a therapeutically effective amount is well within the capacity of persons skilled in the art. The term "effective amount" is intended to include an amount of a blank or drug loaded nanocarrier (i.e., nanoparticle) described herein, e.g., that is effective to treat or prevent a disease or disorder, or to treat the symptoms of the disease or disorder, in a host. Thus, an "effective amount" generally means an amount that provides the desired effect.

The terms "treating", "treat" and "treatment" can include (i) preventing a disease, pathologic or medical condition from occurring (e.g., prophylaxis); (ii) inhibiting the disease, pathologic or medical condition or arresting its development; (iii) relieving the disease, pathologic or medical condition; and/or (iv) diminishing symptoms associated with the disease, pathologic or medical condition. Thus, the terms "treat", "treatment", and "treating" can extend to prophylaxis and can include prevent, prevention, preventing, lowering, stopping or reversing the progression or severity of the condition or symptoms being treated. As such, the term "treatment" can includes both medical, therapeutic, and/or prophylactic administration, as appropriate.

In embodiments, the nanoparticles of the present disclosure may be used to treat a variety of diseases including asthma, COPD, fibrotic lung disease, HIV, cancer, brain tumors, lung tumors, cancers of the blood (e.g., leukemia, myelomas), inflammatory diseases, epilepsy, diabetes, dementia, neurodegenerative disorders, joint disorders, heart disease, infectious diseases, respiratory diseases, hepatic disorders, and autoimmune diseases.

The terms "subject" or "patient" both refer to or mean an individual complex organism, e.g., a human or non-human animal.

The terms "inhibit", "inhibiting", and "inhibition" refer to the slowing, halting, or reversing the growth or progression of a disease, infection, condition, or group of cells. The inhibition can be greater than about 20%, 40%, 60%, 80%, 90%, 95%, or 99%, for example, compared to the growth or progression that occurs in the absence of the treatment or contacting.

The term "therapeutic agent," and similar terms referring to a therapeutic or medicinal function, means that the referenced molecule, macromolecule, drug or other substance can beneficially affect the initiation, course, and/or one or more symptoms of a disease or condition in a subject, and may be used in conjunction with nanoparticles in the manufacture of medicaments for treating a disease or other condition. Suitable therapeutic agents for encapsulation in or absorption on the nanoparticles described herein include hydrophobic therapeutic agents, such as retinoids, such as retinol and esters thereof, and derivatives of retinol, such as all-trans retinoic acid (ATRA) and retinal, small molecules, antibodies, nucleic acids, proteins, hormones, receptors, ligands, cells (e.g., platelet rich plasma (PRP)), growth factors, cell extracts.

The term "therapeutic agent," and similar terms referring to a therapeutic or medicinal function mean that the referenced molecule, macromolecule, drug or other substance can beneficially affect the initiation, course, and/or one or more symptoms of a disease or condition in a subject, and may be used in conjunction with nanoparticles in the manufacture of medicaments for treating a disease or other condition.

Retinol ($C_{20}H_{30}O$; 286.45 g/mol) is a diterpenoid alcohol that has important biological activity. Retinol has a melting point of 61-63 °C, an activity of 3100 units/mg, and a Log P of 6.2. Retinol is practically insoluble in water, is soluble or partly soluble in ethanol, and is miscible with chloroform, ether and petroleum spirits. Retinol is a cosmecutical/therapeutic agent used for various skin conditions including photoaging, acne, wound healing, melasma psoriasis, skin cancer, melanoma and other skin conditions (Orfanos et al., Drug 53:358-388, 1997). Retinol has poor water solubility and poor photostability (Melo et al., J Control Release 138:32-39, 2009; U.S. Patent No. 5,851,538 (Froix et al.). Tretinoin is the carboxylic acid form of vitamin A and is also known as all-trans retinoic acid or ATRA.

Nanoparticles and Preparatory Methods

The disclosure provides nanoparticles that can be formed from a hydrophobic water-insoluble protein such as prolamine, for example, zein and lactoferrin.

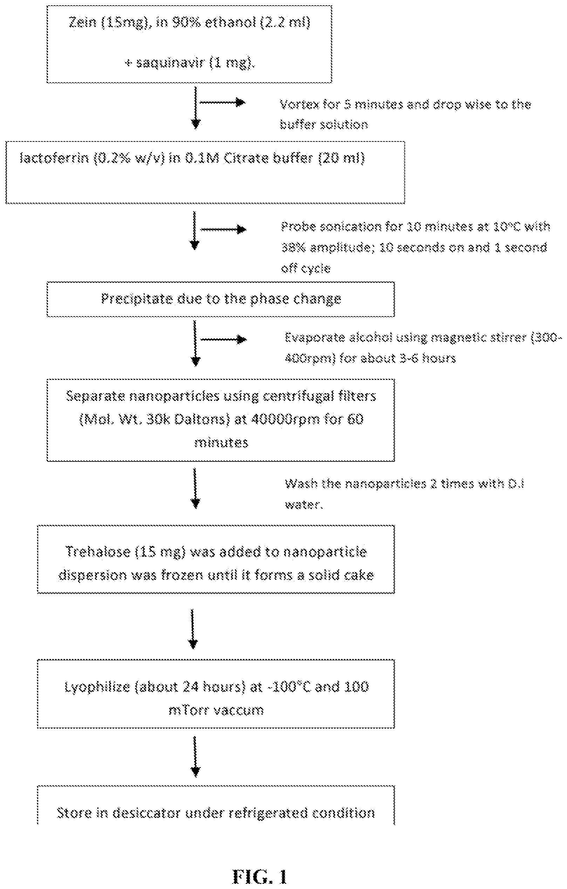

FIG. 1 illustrates by means of a flow chart general steps for preparing zein nanoparticles stabilized by β-casein, not according to the invention. The specific amounts used are for illustration, and many variations can be applied to the procedures described herein, as would be readily recognized by one skilled in the art. In an initial step or phase of the method, a water-insoluble protein (0.4 to 1.25% w/v) is dissolved in a hydroalcoholic solvent (e.g., a combination of ethanol and deionized water). The composition of the solvent may be, for example, 90%:10% v/v or 92%:8% v/v, alcohol to water. For methods where a selected molecule is to be encapsulated in the nanoparticle (e.g., ATRA), the molecule (0.03 to 0.3% w/v) to be encapsulated is added to the solution of this first aqueous phase. The molecule to be encapsulated can be approximately to 50% w/w of the protein polymer.

The pH of the solution can be altered, for example, to bring the pH of the solution to between about pH 6 and about pH 7 by the addition of 0.01N NaOH or 0.01N HC1. If the water pH changes after addition of an acidic molecule, such as retinoic acid, or by a basic molecule, the pH can be readjusted to pH 6-7. The solution of the first phase can be processed, for example, by probe sonication, to aid is the dissolution of the protein.

In a subsequent step of the method, the aqueous solution of the initial step or phase can be added to a β-casein solution containing a buffering agent and an optional natural gums or polysaccharides (e.g., gum arabic). Citrate buffer is suitable buffer. The choice of the buffering agent used for the second aqueous phase is significant for maintaining the pH during nanoparticle formation and for subsequent lyophilization of the formed nanoparticles, as described later in this disclosure. If no buffer is used, or if, for example, 0.1N HCl is used to adjust the pH of the second aqueous phase solution, the particles produced tend to be larger than those produced with the citrate buffer, and the particles tend to demonstrate a wider size range. Use of a citrate buffer produces some of the smallest particle diameter sizes, such as approximately 100 nm. Use of other buffers may produce particles in the same or similar diameter size range of approximately 100 nm to approximately 300 nm, but after the lyophilization step, the average size of the nanoparticles formed using other buffering agents have been know to increase by two to three times.

The pH of the second aqueous phase solution can be adjusted to be between about pH 6.8 and about pH 7.4 to obtain the desired size of nanoparticles. If the pH is outside of this range, the particle size tends to become larger, and the polydispersity index (PDI) of the particles produced becomes higher. The PDI is a measure of the distribution of the particles in different size ranges. The method thus can use the solubility difference of a protein, such as zein, in the hydroalcoholic solution and an aqueous solution with a selected pH of approximately 6.8 to approximately 7.4, close to the isoelectric point of zein (i.e., pl 5 to 9).

The addition of a buffering agent to the second aqueous phase solution may be performed under high ultrasonic shear or under high pressure homogenization, or a combination of both ultrasonic shear and high pressure homogenization. The ultrasonic energy and duration of ultrasonic shear may be particularly significant to the formation of particles in the desired diameter size ranges. The ultrasonic shear energy may be carried out, for example, from 0.6 kW/h to 1.39 kW/h, for a duration of approximately 2 to 10 minutes with a pulse on-time of from 5 to 10 seconds and an off-time of from 1 to 5 seconds. The ultrasonic processing may be significant to the production of particles in the desired size range. When employing high pressure homogenization, the process may be carried out using an orifice size of between 0.1 mm and 0.25 mm, and for a time period of between five to ten minutes at a pressure of from 5000 to 40,000 psi.

After the application of ultrasonic shear or/and high pressure homogenization to the solution of the second phase, the mixture can be stirred to evaporate the ethanol or other solvent to form the nanoparticles. In one embodiment, the stirring can be performed by, for example, a mechanical stirrer, at a rate of from approximately 300 rpm to approximately 500 rpm at room temperature (-23 °C) for approximately one to six hours, or about hours.

The nanoparticles can then be subjected to ultracentrifugal filtration for the purpose of separating the nanoparticles from any residual material. Ultracentifugation may be carried out using centrifugal filters of molecular weight cut-off of about 5 kDa (or other appropriate filters with a higher or lower Mwt cut-off than 5 kDa), and at between 2 kDa and 40 kDa, depending on the encapsulated molecule or drug, or on the particular treatment of the nanoparticles, such as PEGylation. The time of the ultracentrifugation can vary, for example, from about 20 to about 50 minutes. A cryoprotectant may then be added to the nanoparticles. For example, 2% w/v trehalose can be added as a cryoprotectant. Other cryo- or lyoprotectants can also be used, such as sugars, including glucose, sucrose, lactose, ficoll, betaine, or poyols such as mannitol or sorbitol. The nanoparticles can be maintained at, for example, -80 °C to form a solid cake, which can then be lyophilized, such as by drying the nanoparticles in a frozen state under high vacuum. The duration of ultrasonic energy and buffer may be varied according to desired parameters, as would be readily recognized by one skilled in the art.

Accordingly, the range of particle diameter sizes of the nanoparticles described herein can be less than approximately 400 nm, or less than approximately 300 nm. In some embodiments, the range of particle diameter sizes is approximately 100 nm to approximately 300 nm, or approximately 75 nm to approximately 300 nm. While size is discussed in terms of a diameter, the nanoparticles are not necessarily perfectly spherical in shape, although spherical shapes in the nanoparticles can be achieved and can be typical of some embodiments. The dimensions can be measured between opposite sides of the particle, for example, the largest dimension across the particle from opposite sides, or the average of the largest dimension across the particle from opposite sides and the smallest dimension across the particle from opposite sides.

Water-insoluble hydrophobic proteins use for the nanoparticles can be derived from a variety of sources including plant, animal and synthetic sources. In some embodiments, the protein can be from the family of prolamines, which are composed of high amounts of hydrophobic amino acids such as, for example, proline, glutamine and asparagine. These hydrophobic amino acids render the protein water-insoluble. Prolamines can be found in various grains such as corn, wheat, barley, rice, sorghum, and in other plants and animal sources. Some examples of suitable prolamines include, but are not limited to, zein, gliadin, hordein and kafirin.

In some embodiments, white zein can be used to produce suitable nanoparticles, such as those having a diameter of about 100 nm to about 400 nm. Yellow zein can produce particles with relatively larger diameter sizes, and can also produce particles with wider particle diameter size distribution. The pigments in yellow zein may affect the solubility of the yellow zein and nanoparticle formation using yellow zein.

Methods of preparing nanoparticles of a generally smaller diameter size and narrower diameter size range than would otherwise be possible are described herein. These smaller nanoparticles can be prepared by implementing a pH-controlled nanoprecipitation process using one or more particular grades of a base protein, such as zein, and by using various combinations of buffers that are selected to achieve nanoparticle sizes and diameters that render the nanoparticles non-immunogenic.

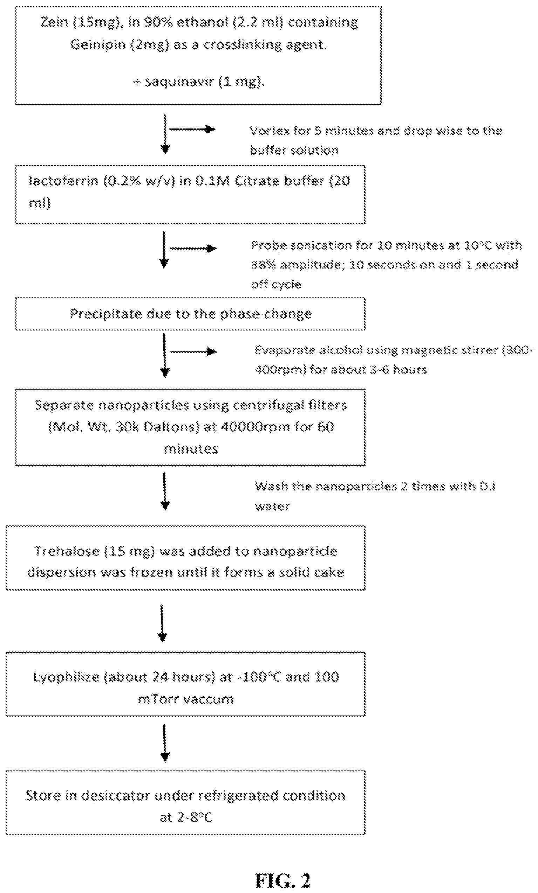

FIG. 2 illustrates by means of a flow chart general steps for preparing genipin cross-linked zein-casein nanoparticles, not according to the invention.

Zein-casein nanoparticles may be cross-linked by adding genipin (1.0mg/mL) as crosslinking agent to the organic phase and following the previous steps as outlined above (see e.g., FIG. 1). Subsequently, the resulting nanoparticles may be purified, washed and lyophilized.

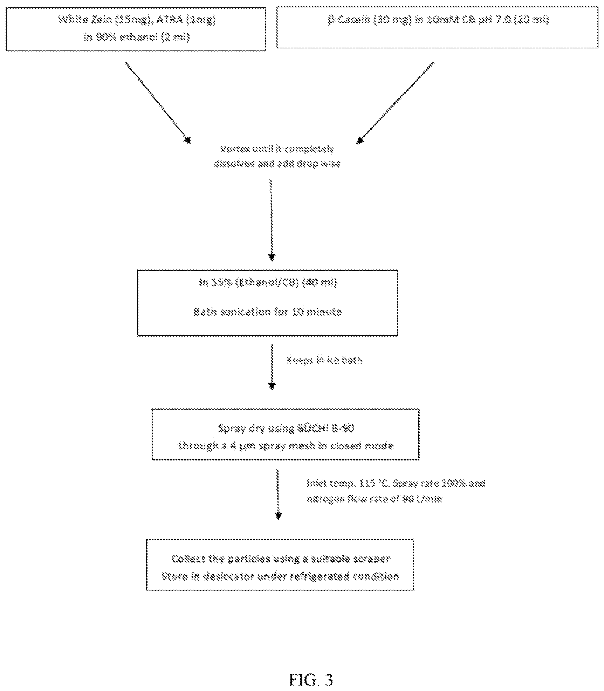

As shown in FIG. 3, in embodiments, nanoparticles may be prepared by spray drying. In embodiments not according to the invention, spray drying process may be carried out by dissolving both zein and casein in binary ethanolic solution (55% Ethanol/CB) so that the total concentration of proteins is about 1%wt.

A cargo molecule (e.g., a BCS class II/IV drug) may be added from an ethanolic stock solution at a concentration of about 10% (w/v) to the suspension prior to spray drying. The spray drying may be performed using new generation spray-dryer, e.g., Nano Spray Dryer B-90, BÜCHI, at room temperature for about 6 hours. The operating parameters may be set as follows: spray drying through a 4 $\mu$m spray mesh, spray rate of about 100%, the inlet temperature was set to about 100°C and nitrogen flow rate of about 150 L/min.

For PEG-zein, the solution may be bath sonicated and visually examined before spraying to ensure complete solubility. Dry particles may be collected from a collecting drum using a suitable scraper and stored in a desiccator until used.

In embodiments, after spray drying, the zein-casein nanoparticles may be cross-linked with genipin. For example, after spray drying the resulting zein-casein nanoparticles may be incubated in an appropriate buffer (e.g., citrate buffer) solution at a pH of about 7 containing genipin (1.0mg/mL) for about 4 hours at room temperature. The resulting nanoparticles may be purified using Millipore centrifugal filters (10k MWCO) and washed with deionized $H_2O$. Finally, a cryoprotectant (e.g., trehalose) may added and then the particles may be cooled to about -80°C followed by lyophilization for about 48 hours at about -105°C/100 mTorr vacuum. The lyophilized particles may then be stored in a desiccator.

The nanoparticles can be prepared with a wide variety of "cargo" or "cargo molecules". For example, particles or agents, having varying physicochemical properties, can be added in the preparation of the protein nanoparticles to provide encapsulated, adsorbed, complexed and/or conjugated materials with the nanoparticles. The particles can entrap small hydrophilic molecules, small hydrophobic molecules, and/or macromolecules. An encapsulation efficiency of approximately 60% to approximately 80% or greater can be achieved. The nanoparticles can provide sustained delivery of the encapsulated molecule one to seven days, or one to two weeks, in an in vitro or in vivo environment. In some embodiments (e.g., proteins/antibodies and the like), cargo may be adsorbed/complexed/conjugated to the surface of the nanoparticle.

In embodiments, cargo or cargo molecules are pharmaceutical materials. Such materials which are suitable for use with the present nanoparticles as encapsulated cargo or cargo molecules, complexed or conjugated cargo molecules or adsorbed cargo molecules include any materials for *in vivo* or *in vitro* use for diagnostic or therapeutic treatment of a subject which can be associated with the nanoparticle without appreciably disturbing the physical integrity of the nanoparticle.

In other embodiments, the cargo or cargo molecules are agricultural materials. Such materials which are suitable for use with the nanoparticles as described herein include any materials for *in vivo* or *in vitro* treatment, diagnosis, or application to plants or non- mammals (including microorganisms) which can be associated (i.e., encapsulated, conjugated or adsorbed) with the nanoparticles without appreciably disturbing the physical integrity of the nanoparticles.

In another embodiment, the cargo or cargo molecules are immuno-potentiating agents. Such materials which are suitable for use with the nanoparticles as described include any antigen, hapten, organic moiety or organic or inorganic compounds which will raise an immuno-response which can be associated with (i.e., encapsulated, conjugated or adsorbed) the nanoparticles without appreciably disturbing the physical integrity of the nanoparticles. The nanoparticles may be useful for production of antivirals for the treatment of diseases such as AIDS.

These nanoparticles may be used in a variety of *in vivo*, *ex vivo* or *in vitro* diagnostic or therapeutic applications. Some examples are the treatment of diseases such as cancer, autoimmune disease, genetic defects, central nervous system disorders, infectious diseases and cardiac disorders, diagnostic uses such as radioimmunossays, electron microscopy, PCR, enzyme linked immunoadsorbent assays, nuclear magnetic resonance spectroscopy, contrast imaging, immunoscintography, and delivering pesticides, such as herbicides, fungicides, repellants, attractants, antimicrobials or other toxins. Non-genetic materials are also included such as growth factors, hormones, chemokines, cytokines, interleukins, interferons, tumor necrosis factor, granulocyte colony stimulating factor, and other protein or fragments of any of these, antiviral agents.

The invention also provides for nanoparticles as oral delivery devices, such as nanoparticles containing an active agent (drug). The nanoparticles may provide targeted delivery and temporal control of the release of the agent. The agent may be, for example, an agent effective to treat childhood maladies, for example, retinol or retinoic acid, and the like among other agents described herein.

The invention also provides a kit for the preparation of nanoparticles described herein.

The invention therefore provides nanoparticles encapsulating various agents. In one embodiment, the method can be for producing non-immunogenic nanoparticles. A method not according to the invention can include providing a hydrophobic water-insoluble protein; dissolving the protein with a hydroalcoholic solvent and a cargo molecule to provide a first aqueous phase solution; adding the hydroalcoholic solution to buffering agent containing beta-casein (and an optional natural gum or polysaccharide) to produce a second aqueous phase solution having a pH of between approximately pH 6.8 and approximately pH 7.4; processing the second aqueous phase solution to effect a reduction in diameter size of particles within the dispersion; evaporating any residual solvent to produce nanoparticles having a diameter size of less than approximately 400 nm. The nanoparticles may then be centrifuges for isolation and collection. Alternatively, in embodiments, the nanoparticles may be cross-linked or spray dried.

In embodiments, the selection of the organic solvent or mixtures of organic solvents for the solubilisation of Zein prior to spray drying may include one or more of the following: water; EtOH/water; EtOH/citrate buffer, pH about 7.4; EtOH/phosphate buffered saline, pH about 7.4; IPA/water; MeOH/water; Acetone/water and DCM/Ethanol (1:1). In embodiments, several organic mixtures may be used to conduct the spraying process. In one embodiment, the final organic solvent/aqueous ratio is about 3:2 in final volume of 40 ml.

In embodiments, spray dried zein may be stabilized with one or more molecules including citric acid, SLS, Tween 80, Pluronic F68, Lecithin, Lecithin-Pluronic F68, PVP (polyvinyl pyrrolidone), PEG (polyethylene glycol) 20 kDa, TPGS 1000, Gum Arabic, Casein sodium salt, β-Casein, Dextran, PSA (polysialic acid).

In embodiments, pectins and gums may be added to the material matrix, where the total concentration in the spray solution is about 1% wt.

The method as disclosed herein may include lyophilizing the nanoparticles following centrifugation. The method may further include storing the nanoparticles under conditions that restrict exposure of the nanoparticles to atmospheric pressure. The base protein may be, for example, a selected grade of zein, such as white zein.

The buffering agent may be a citrate buffer. The processing of the second aqueous phase solution to effect a reduction in diameter size of particles can further include subjecting the nanoparticles to ultrasonic shear, high pressure homogenization, or a combination thereof. For other nanoparticle preparations, for example, surfactants may be absent or other surfactants may be used (e.g., where sodium lauryl sulfate is used in addition to non-ionic surfactants to prepare zein nanoparticles).

The method may include adding to the protein in the formation of the first phase solution a molecule for nanoparticle encapsulation. The molecule may be a therapeutic substance selected for administration to a subject, to provide a therapeutically-active, non-immunogenic nanoparticle. The protein may also be PEGylated and/or cross-linked.

The invention further provides a therapeutic composition comprising a non-immunogenic nanoparticle formed by the encapsulation of a therapeutic molecule in a hydrophobic, water insoluble protein, the nanoparticle having a diameter of less than about 400 nm. In some embodiments, the diameter of the particles is about 100 nm to about 400 nm, or about 100 nm to about 300 nm. The invention also provides a pharmacologically therapeutic amount of a non-immunogenic nanoparticles comprising a therapeutic agent, the nanoparticles having average diameters of less than about 400 nm. The nanoparticles may be used for the manufacture of a medicament for use in the treatment of a disease or condition in a subject suffering from, or at risk of suffering from, the disease or condition that may be treated by the therapeutic agent (i.e., in need thereof).

Variations of Protein, Polymer, and Nanoparticle Components

Variations of the zein nanoparticles described herein may also be prepared. For example, in place of zein, other hydrophobic prolamine proteins, such as gliadin, hordein and kafirin may be used as the protein for nanoparticle formation. Accordingly, gliadin nanoparticles, hordein nanoparticles, and kafirin nanoparticles may be prepared and used similar to the zein nanoparticles described herein.

Additionally, the protein of the nanoparticles may be conjugated to moieties such as PEG to modify the surface of the nanoparticles. The surface modifying moiety may be PEG moieties or other water soluble polymers, such as polyvinylpyrrolidone (PVP), polyglycolic acid (PGA), polyvinyl alcohol (PVA), chitosan, dextran, polyethyleneimine (PEI), polysialic acid (PSA), polyacrylic acid (PAA), and the like. These water soluble polymers may be conjugated to any of the hydrophobic prolamine proteins, such as zein, gliadin, hordein and kafirin, to form surface modifications of the nanoparticles.

Similarly, hydrophobic polymers can be complexed, mixed or conjugated to a prolamine nanoparticle. Such polymers can include, for example, polycaprolactone, poly lactic acid-co glycolic acid, polypropylene oxide, polyaspartate, polygultamate, spermine, polylysine, polyethylene imine or polyacrylates (for example, polymethacrylate, polydimethylamino ethyl acrylate, and the like). Natural polymers can also be complexed, mixed or conjugated to prolamine nanoparticle such as other protein polymers (albumin, caesin, gelatin, and the like), and carbohydrate polymers such as chitosan, dextran, gum Arabica, dextran-grafted casein, alginates or combinations thereof. Likewise, fatty acids can also be mixed, complexed or conjugated to a prolamine nanoparticles surface. Examples of such fatty acids can include stearic acid, palmitic acid, phosphatidyl ethanolamine, and/or oleic acid. These polymers and/or fatty acids can be conjugated to any of the hydrophobic prolamine proteins, such as zein, gliadin, hordein and kafirin, to form surface modified nanoparticles.

Because zein and beta-casein are proteins, a further advantage of using these molecules in formation of nanoparticles is realized in that proteins have a large number of surface functional groups that may be used to attach targeting ligands, imaging agents, drugs and other polymers for drug targeting to specific tissues and other biomedical applications. Other or further modifications may be made to the prolamine hydrophobic core or to the nanoparticle surfaces. These may include conjugating stimuli responsive elements, such as polyhydroxyethylmethacrylate, to the nanoparticles to prepare pH sensitive nanoparticles or poly (N-isopropylacrylamide) to prepare thermosensitive nanoparticles. In addition, the prolamine nanoparticles may be cross-linked, for example, using cross-linkers such as glutaraldehyde, genipin, citric acid, polysialic acid (PSA), and the like, to control drug release and increase drug encapsulation yield and efficiency.

Pharmaceutical Formulations of Nanoparticles

The nanoparticles described herein may be used to prepare therapeutic pharmaceutical compositions. The nanoparticles may be added to the compositions in the form of an aqueous dispersion or as a dry powder of lyophilized nanoparticles. The nanoparticles may be formulated as pharmaceutical compositions and administered to a mammalian host, such as a human patient, in a variety of forms. The forms can be specifically adapted to a chosen route of administration, such as oral administration.

The nanoparticles described herein may be orally administered in combination with a pharmaceutically acceptable vehicle, such as an inert diluent. The weight percentage of agent in the compositions and preparations may vary and may also conveniently be from about 2% to about 60% of the weight of a given unit dosage form. The amount of active compound in such therapeutically useful compositions containing nanoparticles is such that an effective dosage level can be obtained. Dispersions, aerosol formulations, gels, and the like may also contain one or more of the following: binders such as gum tragacanth, acacia, corn starch or gelatin. A unit dosage form, in addition to materials of the above type, may include a liquid carrier, such as a vegetable oil or a polyethylene glycol. Various other materials may be present to modify the physical form a unit dosage form. A topical formulation may contain the nanoparticles, in addition to methyl and propyl parabens as preservatives, and optionally a dye to add color. Any material used in preparing a unit dosage form should be pharmaceutically acceptable and substantially non-toxic in the amounts employed. In addition, the nanoparticles dispersion or lyophilized nanoparticles may be incorporated into additional sustained-release preparations and devices.

Dispersions of the nanoparticles can be prepared in water, optionally mixed with a buffer, or in other pharmaceutically acceptable solvents, or mixtures thereof. Under ordinary conditions of storage and use, preparations may contain a preservative to prevent the growth of microorganisms. The ultimate dosage form should be sterile, fluid and stable under the conditions of manufacture and storage. The liquid carrier or vehicle can be a liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The prevention of the action of microorganisms can be brought about by various antibacterial and antifungal agents, for example, parabens, chlorobutanol, phenol, sorbic acid, thiomersal, and the like. In many cases, it will be preferable to include isotonic agents, for example, sugars, buffers, or sodium chloride in some formulations.

Sterile solutions can be prepared by incorporating the nanoparticles in the required amount in an appropriate solvent with various of the other ingredients enumerated above, as required, followed by filter sterilization. In the case of sterile powders for the preparation of sterile solutions, methods of preparation may include vacuum drying and freeze drying techniques, which yield a powder of the nanoparticles plus any additional desired ingredient present in the previously sterile-filtered material.

In embodiments, compounds described herein may be formulated for oral administration. Compounds described herein may be formulated by combining the active compounds with, e.g., pharmaceutically acceptable carriers or excipients. In various embodiments, the compounds described herein may be formulated in oral dosage forms that include, by way of example only, tablets, powders, pills, dragees, capsules, liquids, gels, syrups, elixirs, slurries, suspensions and the like.

In embodiments, pharmaceutical preparations for oral use may be obtained by mixing one or more solid excipients with one or more of the compounds described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In embodiments, disintegrating agents are optionally added. Disintegrating agents include, by way of example only, cross-linked croscarmellose sodium, polyvinylpyrrolidone, agar, or alginic acid or a salt thereof such as sodium alginate.

In embodiments, dosage forms, such as dragee cores and tablets, are provided with one or more suitable coating. In embodiments, concentrated sugar solutions are used for coating the dosage form. The sugar solutions, optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

In embodiments, therapeutically effective amounts of at least one of the compounds described herein may be formulated into other oral dosage forms. Oral dosage forms include push-fit capsules made of gelatin, as well as soft, sealed capsules made of gelatin and a plasticizer, such as glycerol or sorbitol. In embodiments, push-fit capsules contain the active ingredients in admixture with one or more filler. Fillers include, by way of example only, lactose, hinders such as starches, and/or lubricants such as talc or magnesium stearate and, optionally, stabilizers. In embodiments, soft capsules, contain one or more active compound that is dissolved or suspended in a suitable liquid. Suitable liquids include, by way of example only, one or more fatty oil, liquid paraffin, or liquid polyethylene glycol. In addition, stabilizers may be optionally added.

In embodiments, therapeutically effective amounts of at least one of the compounds described herein are formulated for buccal or sublingual administration. Formulations suitable for buccal or sublingual administration include, by way of example only, tablets, lozenges, or gels.

Useful dosages of drug loaded nanoparticles described herein can be determined by comparing their in vitro activity, and in vivo activity in mammalian animal models. A mammal includes a primate, human, rodent, canine, feline, bovine, ovine, equine, swine, caprine, bovine and the like.

Methods for the extrapolation of effective dosages in mice, and other animals, to humans are known to the art; for example, see U.S. Patent No. 4,938,949 (Borch et al.).The amount of a compound, or an active salt, prodrug, or derivative thereof, loaded into a nanoparticle required for use in treatment may vary not only with the particular compound or salt selected but also with the route of administration, the nature of the condition being treated, and the age and condition of the patient, and will be ultimately at the discretion of an attendant physician or clinician.

The therapeutic agent loaded nanoparticle can be conveniently administered in a unit dosage form, for example, containing 5 to 1000 mg/m$^2$, conveniently 10 to 750 mg/m$^2$, most conveniently, 50 to 500 mg/m$^2$ of active ingredient per unit dosage form. The desired dose may conveniently be presented in a single dose or as divided doses administered at appropriate intervals, for example, as two, three, four or more sub-doses per day. The subdose itself may be further divided, e.g., into a number of discrete loosely spaced administrations.

## EXAMPLES

Example 1. Materials and Methods for Zein-Casein Nanoparticles (not according to the invention).

Phase separation method.

15 mg of white zein form corn (Grade F6000) was dissolved in 2 ml of 90% EtOH so that the total concentration was 0.5% (w/v). To this ethanolic solution, all trans retinoic acid (ATRA) was added from a stock solution at an optimum concentration of 1 mg. The organic phase was added drop wise to 0.1 M citrate buffer solution containing 0.15% (w/v) beta-casein (Sigma Cat # C6905) under probe sonication. Optionally a stabilizer (e.g., gum arabic at 0.1% w/v) may be added during this step or added to the 0.1 M citrate buffer prior to drop wise addition of organic phase. The probe sonication was set to 38% amplitude for 10 min (10 sec on and 1 sec off cycle). The zein-casein dispersion was left under a magnetic stirrer (300 rpm) for 4 hours to evaporate the EtOH. Further, the nanoparticles were separated using Millipore centrifugal filters (MWCO 5-10kDa; 40,000 rpm for 60 minutes) and washed several times with DI H$_2$O using a pipette and/or PBS, pH 7.0. Finally, a cryoprotectant (trehalose) was added and then placed in -80°C followed by lyophilization for 48 hours at -105°C/100 mTorr vacuum. The formulation was then stored in closed vials in a dessicator (at 2-8°C) until further use.

For genipin crosslinked zein-casein nanoparticles, genipin was added (1.0 mg/ml) to the organic phase and the remaining steps were followed as above. Subsequently, the resulting nanoparticles were purified, washed and lyophilized.

Spray drying method for ZC nanoparticles.

Spray drying process was carried out by dissolving both zein and casein in binary ethanolic solution (55% EtOH/citrate buffer) so that the total concentration of proteins was 1%. For PEG-zein, the solution was bath sonicated and visually examined before spraying to ensure complete solubility. ATRA was added from ethanolic stock solution in a concentration of 10% to the suspension prior to spray drying. The spray drying was performed using a Nano Spray Dryer B-90 BÜCHI at room temperature for 6 hours. The operating parameters were set as follows: Spray drying through a 4 um spray mesh, spray rate 100%, the inlet temperature was set to 100°C and nitrogen flow rate of 150L/min.

Dry particles were collected from the collecting drum using a suitable scraper and stored in a dessicator until use.

For genipin crosslinked ZC nanoparticles, after spray drying, the ZC nanoparticles were crosslinked by incubation in a citrate buffer solution (pH 7) containing genipin (1.0 mg/ml) for 4 hours at room temperature. The resulting nanoparticles were purified using Millipore centrifuge filters (10 kDa MWCO) and washed with DI water. Finally, cryoprotectant (trehalose) was added and then the nanoparticles were placed in -80°C followed by lyophilization for 48 hours at -105°C/100 mTorr vacuum. The lyophilized nanoparticles were stored in a dessicator until use.

Particle size analysis and zeta potential was measured using Dynamic Light Scattering (DLS). The average size as well as zeta potential of the particles were measured using a Malvern Zetasizer-S 3600 (Malvern Instruments Inc., South Borough, MA). For atomic force microscopy, 100 $\mu$l of the dispersion was placed on a polyethylene amine coated glass cover slip and air dried. AFM images of ZC nanoparticles were characterized using Agilent 5500 AFM/SPM microscope. AFM images were collected using the scan area of 1 um. For scanning electron microscopy (SEM) ZC nanoparticles were dispersed in water at a concentration of 20 $\mu$g/ml and applied on a polished sample grid. The samples were vacuumdried and metallized with a 3 nm gold layer.

The ATRA amount was determined by reverse phase HPLC. Briefly 2 mg of ATRA loaded ZC nanoparticles were dispersed in DI water then centrifuged at 14,000 rpm for 10 min. The supernatant was discarded and the remaining pellet was dissolved in 50% hydroalcoholic solution/citrate buffer, probe sonicated for 5 min followed by bath sonication for 5 min. The drug concentration was determined form the calibration curve. The loading and encapsulation efficiency were calculated as follows:

$$DL\% = (\text{weight of ATRA in ZC nanoparticles}/\text{weight of ZC nanoparticles}) * 100$$

$$EE\% = (\text{weight of ATRA in ZC nanoparticles}/\text{weight of the feeding ATRA}) * 100$$

Column: C18 column, 100Å, 5 um, 4.6 mm x 150 mm; Mobile phase: acetonitrile (1.13 ml/min)-1% w/v ammonium acetate buffer (0.12 ml/min); Detection wavelength: 340 nm; Injection volume: 50 $\mu$l; Run time: 10 min.

Loading efficiency (ATRA) = 1.17-4.1%
Encapsulation efficiency (ATRA) = 61-88.56%

For Nile Red ZC nanoparticles:

Loading efficiency = 2.9%
Encapsulation efficiency = 71.06%

For curcumin ZC nanoparticles:

Loading efficiency = 0.98%
Encapsulation efficiency = 73.5%

Optimization

To optimize the formulation parameters, a three level Box-Behnken design was used. A total of 15 experiments with three formulation variables were used. The three parameters were studied at three levels (low, medium and high). The dependent variables that were selected for study: particle size (Y1), PDI (Y2), and % EE (Y3). See Table 1.

Table 1. Optimization of the formulation parameters.

| Coded value | Actual values | | |
|---|---|---|---|
| | $X_1$ | $X_2$ | $X_3$ |
| High (+1) | 3:2 | 9 | 90% |
| Medium (0) | 1:2 | 7,4 | 70% |
| Low (-1) | 0.5:2 | 5 | 55% |
| $X_1$, zein/casein ratio; $X_2$, pH of aqueous phase; $X_3$ % hydroalcoholic phase | | | |

Statistical analysis

The effect of various parameters were analyzed using multiple linear regression analysis and ANOVA at a significance level of $p < 005$ (MINITAB™ software).

The physical state of ATRA in the formulation was determined by differential scanning calorimeter DSC Q200 (TA Instruments, Inc. USA). Accurately weighed samples (5 mg) were crimped into aluminum crimp pans (T Zero Lid #T100819) and heated at a rate of 10°C/min from 23 to 300°C under nitrogen atmosphere (flow rate 20 ml/min). A blank aluminum cell was used as a reference. The thermograms were processed using TA Universal Analysis software (TA Instruments Inc, USA).

In vitro degradation and release of ATRA from ZC nanoparticles

The degradation profile of ZC nanoparticles was determined by incubating the zein-casein nanoparticles in simulated gastric fluid and intestinal fluid for up to 4 hours. The samples were analyzed by gel electrophoresis. Further, ATRA release in simulated gastric fluid was quantified by HPLC. Briefly, 10 mg of ATRA loaded ZC nanoparticles was suspended in 1.5 ml Eppendorf tubes containing release medium. The tubes were place in a shaker at 37°C. At different time points, the samples were removed and centrifuged at 14,000 rpm for 10 min. the supernatant was carefully removed using a 1 ml pipette and the remaining pellet was dissolved in 50% EtOH. The concentration of ATRA was determined using HPLC as described.

Stability studies

For solid state stability of ATRA loaded zein-casein nanoparticles, 40 mg of ATRA loaded nanoparticles was kept in six sealed glass vials and maintained in the dark at room temperature (i.e., 25°C) as well as at 4°C in a refrigerator. At different time points, particle size, PDI, encapsulation efficiency and percent drug remaining in the nanoparticles were determined.

Hybrid core-shell particles were formed during the phase separation process. The particles were uniform and with an average particle size of 90-150 nm, having a narrow polydispersity (PD). The nanoparticles had a high negative zeta potential indicating good colloidal stability. (See Tables 2 and 3.)

Table 2. Nanoprecipitation (Phase separation) Method (from third pdf)

|  | Size (nm) | PDI | Zeta Potential (mV) |
|---|---|---|---|
| ZC nanoparticles | 91.19±1.64 | 0.105±0.042 | -44.8±2.9 |
| Genipin Crosslinked ZC | 101.2±4.3 | 0.118±0.09 | -52±1.6 |
| ZCG | 121.5±3.67 | 0.103±0.021 | -36.6±4.3 |
| Nile Red loaded ZC | 115.2±7.54 | 0.143±0.057 | -38.6±1.8 |
| ATRA loaded ZC | 135.7±11.2 | 0.239±0.069 | -40.5±3.4 |
| ATRA loaded ZCG | 144.5±5.21 | 0.291±0.007 | -38.9±7.8 |
| ZC, zein-casein nanoparticles; ZCG, zein-casein gum arabic nanoparticles; ATRA, all trans retinoic acid. | | | |

Table 3. Particle characteristics and encapsulation efficiency of blank and ATRA loaded ZC particles.

| Formulation | Size (nm) | PDI | Zeta Potential (mV) | LE% | EE% |
|---|---|---|---|---|---|
| ZC | 91.91±1.64 | 0.105±0.042 | -44.8±2.9 | - | - |
| ATRA loaded ZC | 135.7±11.2 | 0.239±0.069 | -40.5±3.4 | 4.1±0.52 | 88.56±26 |

Based on the Box-Behnken model, the formulation was optimized on the basis of observed and predicted values. The optimal factors were determined as zein/casein ratio (1:2), pH of the aqueous phase (7.4) and % of hydroalcoholic phase (70%).

The ATRA was encapsulated in zein-casein nanoparticles with high encapsulation efficiency (see Table 3). Chemical stability studies indicated that ATRA loaded ZC nanoparticles are stable in storage conditions and that there was no remarkable change in the particle size nor ATRA content for up to 60 days. In vitro degradation and release study showed that the nanoparticle formulation exhibited a sustained release profile. Further, the ZC nanoparticles were stable in the simulated gastric fluid. (May have to use flix).

Differential Scanning Calorimetry (DSC).

The physical state of the drug in the formulation was determined by DSC in a DSC Q200 (TA Instruments Inc., USA). DSC thermograms (not shown) were obtained for zein blank, ATRA loaded particles and pure ATRA. Accurately weighed samples (5 mg) were crimped into aluminum crimp pans (T Zero Lid #T100819) and heated at a rate of 10°C/min from 23 to 300°C under a nitrogen atmosphere (flow rate 20 ml/min). A blank aluminum cell was used as a reference. The thermorgrams (not shown) were processed using TA Universal Analysis software (TA Instruments, Inc. USA). The thermograms obtained from the blank formulations, pure drug and drug loaded formulations were compared to each other to determine the physical state of ATRA in the formulation.

Example 2. Influence of solvents.

To identify suitable solvents and to stabilize the nanoparticles using a nanospray dryer, a model hydrophobic compound was used (curcumin) as an encapsulant in zein nanoparticles.

As shown in Table 4, various spray drying solvents had different effects on the zein nanoparticles, including effects on size, PDI and zeta potential.

Table 4. Affects of various solvents on nanoparticle characteristics.

| Solvents | Class | PS | PDI | Zeta potential | Observation |
|---|---|---|---|---|---|
| Water | - | - | - | - | Not Soluble |
| EtOH/water (2:1) | Class 3 | 4036 | 1 | -5.2 | Aggregates |
| EtOH/CB 7.4 (2:1) | Class 3 | 664 | 0.557 | -8.82 | Aggregates |
| EtOH/PBS 7.4 (2:1) | Class 3 | 851.5 | 0.694 | -6.02 | Aggregates |
| IPA/water (2:1) | Class 3 | 853 | 0.682 | -6.67 | Aggregates |
| MeOH/water (2:1) | Class 2 | 1200 | 1 | -11 | Aggregates |
| Acetone/water (2:1) | Class 3 | 3190 | 1 | -16.3 | Aggregates |
| DCM/EtOH (1:1) | Class 2 | 920 | 0.81 | -9.3 | Aggregates |

The selection of the organic solvent or mixtures of organic solvents was initially considered for the solubilization of zein prior to spray drying. Several organic mixtures were used to conduct the spraying process. The final organic solvent/aqueous ratio was 3:2 for a final volume of 40 ml.

Example 3. Influence of stabilizers

As shown in Table 5, various stabilizers used in spray drying had different effects on the zein nanoparticles, including effects on size, PDI, zeta potential and aggregation characteristics.

Table 5. Affects of stabilizers on nanoparticle characteristics.

| Materials (1:1 ratio in EtOH aq) | PS | PDI | Zeta potential | Observation |
|---|---|---|---|---|
| None | 4036 | 1 | -5.2 | Aggregates |
| Citric acid | 664 | 0.557 | -8.82 | Aggregates |
| SLS | 742 | 0.727 | -27 | Transparent |
| Tween 80 (1% v/v) | 2293 | 0.881 | -22 | Sticky |
| Tween 80 (0.1% v/v) | 2613 | 1 | -34.8 | Aggregates |
| Pluronic F68 | 1575 | 1 | -29.1 | Aggregates |
| Lecithin | - | - | - | Sticky |
| Lecithin-Pluronic | 958.5 | 0.863 | -23 | Aggregates |
| PVP | 7273 | 1 | -13.9 | Aggregates |
| PEG 20 kDa | 548 | 0.572 | -15 | Aggregates |
| TPGS 1000 | 6636 | 1 | -25.3 | Aggregates |
| Gum arabic | 4612 | 1 | -17 | Aggregates |
| Casein sodium salt | 181.3 | 0.69 | -38 | Transparent |
| Beta-casein | 114.9 | 0.304 | -42.1 | Transparent |
| Dextran | 1314 | 0.896 | -15 | Aggregates |
| PSA | 3127 | 1 | -18.8 | Aggregates |
| SLS, sodium lauryl sulphate; PVP, polyvinylpyrrolidine; PSA, polysialic acid. | | | | |

Zein and stabilizer in a ratio of 1:1 were added in an approximate volume of 60% EtOH (i.e., the total concentration

17

of the material matrix in the organic solution was kept constant at 1%) and then bath sonicated at room temperature until all of the components were completely dissolved.

Example 4. Spray dried particles, physiochemical characterization.

Characterization of particle size, distribution and zeta potential were determined by dynamic laser light scattering using the Malvern Zetasizer S3600 (Malvern Instruments Inc., Southborough, MA). See, e.g., Table 6 and FIG..

For spray dried beta casein nanoparticles, the following data was generated:

|  |  | Size (d.nm) | %Intensity | Width (d.nm) |
|---|---|---|---|---|
| Z average (d.nm): 91.46 | Peak 1 : | 102.0 | 98.2 | 40.37 |
| : 0.179 | Peak 2: | 4813 | 1.8 | 718.4 |
| Intercept: 0.961 | Peak 3: | 0.000 | 0.0 | 0.000 |
| Resulting quality: Good |  |  |  |  |

Table 6. Properties for spray dried ZC nanoparticles (not according to the invention)

|  | Size (nm) | PDI | Zeta Potential (mV) |
|---|---|---|---|
| Beta-casein | 91.46 | 0.179 | -42.5 |
| ZC | 186.7±8.97 | 0.280±0.064 | -31±7.15 |
| ATRA loaded ZC | 198.2±12 | 0.295±0.088 | -35±4.91 |
| ZC, zein casein nanoparticles; ATRA, all trans retinoic acid. |  |  |  |

Example 5. Spray dried zein-casein (ZC) nanoparticles (not according to the invention) with natural gums or polysaccharides.

Pectin and gums was added to the material matrix (zein and casein) so the total concentration in the spray solution was about 1% wt. The spray drying process was performed as mentioned above. To investigate the stability of ZC nanoparticles with pectin and gums, the size, PDI, and zeta potential were measured in acidic pH (1.5) as well as in water (neutral pH). See tables 7, 8, and 9.

Table 7. Snrav dried ZC nanoparticles with gum arabic (ZCG)

|  | Size (nm) | PDI | Zeta potential (mV) |
|---|---|---|---|
| pH 1.5 | 200 | 0.471 | 14.4 |
| Water | 237 | 0.377 | -42.2 |

Table 8. Spray dried ZC nanoparticles with high methoxyl pectin (HMP)

|  | Size (mn) | PDI | Zeta potential (mV) |
|---|---|---|---|
| pH 1.5 | 239.6 | 0.470 | -0.175 |
| Water | 236.3 | 0.367 | -44.2 |

Table 9. Spray dried ZC nanoparticles with low methoxyl pectin (LMP)

|  | Size (nm) | PDI | Zeta potential (mV) |
|---|---|---|---|
| pH 1.5 | 182.1 | 0.469 | 0.09 |
| Water | 219.7 | 0.367 | -37 |

Example 6. Characterization of spray dried PEG-Zein nanoparticles, including comparisons with zein-casein nanoparticles (not according to the invention).

Table 10 shows size, PDI, and zeta potential data for various pegylated-Zein combinations, with PEGs of various molecular weights (5-20 kDa) between spray dried and non-spray dried processes.

Table 10. Affects of PEG molecular weight on nanoparticle characteristics.

| Zein PEGylation | Size (nm) | PDI | Zeta potential (mV) |
|---|---|---|---|
| Peg-Zein 5 kDa | 69.75±1.48 | 0.275±0.04 | +8.33 |
| Spray dried PEG-Zein 5 kDa | 102.3±61.2 | 0.326±0.133 | -1.21 |
| Peg-Zein 10 kDa | 98.7±13.67 | 0.233±0.014 | +4.84 |
| Spray dried PEG-Zein 10 kDa | 78.62±12.2 | 0.201±0.03 | +1.73 |
| PEG-Zein 20 kDa | Aggregates | | |
| Spray dried PEG-Zein 20 kDa | 335.3 | 0.947 | - |

Physical mixtures were also examined (see Table 11).

Table 11. Physical mixtures of PEG and zein under spray drying conditions.

| Physical mixture | Size (nm) | PDI | Zeta potential (mV) |
|---|---|---|---|
| Spray dried PEG 10 kDa + Zein | Aggregates | Aggregates | -7(aggregates) |
| Spray dried PEG 20 kDa + Zein | 1548 | 1 | -15 (aggregates) |

As can be seen between Tables 10 and 11, PEGylated zein has quite different properties from simple PEG + zein mixtures.

Table 12 demonstrates the stability of various zein nanoparticle compositions in simulated gastric fluid (SGF, pH 1.3).

Table 12. Affects of SGF on stability of various nanoparticle's characteristics.

| | Neutral pH | | Acidic pH | |
|---|---|---|---|---|
| | Size (nm) | PDI | Size (nm) | PDI |
| β-Casein | 210.4 | 0.212 | Aggregates | |
| ZC | 98.4 | 0.17 | 182.8 | 0.303 |
| ZCG | 1233.6 | 0.077 | 163.4 | 0.151 |
| PEG-Z | 81.03 | 0.231 | 67.88 | 0.187 |
| Spray dried PEG-Z | 129.2 | 0.425 | 72.41 | 0.221 |
| ZCG, zein-casein gum arabic nanoparticles. | | | | |

Characteristics for ATRA loaded zein casein nanoparticles may be seen in Tables 13 (prepared by phase separation) and 14 (prepared by spray drying).

Table 13. Phase separation preparations

| | Size (nm) | PDI | Zeta Potential |
|---|---|---|---|
| ZC | 91.19±1.64 | 0.105±0.042 | -44.8±2.9 |
| ATRA loaded ZC | 135.7±11.2 | 0.239±0.069 | -45.0±3.4 |

Table 14. Spray drying preparations.

|  | Size (nm) | PDI | Zeta Potential |
|---|---|---|---|
| ZC | 186.7±8.97 | 0.280±0.064 | -31±7.15 |
| ATRA ZC | 198.2±12 | 0.295±0.088 | -35±4.91 |

Table 15 shows that effect of spray drying on ATRA loaded zein-PEG nanoparticles using various ATRA concentrations.

Table 15. Concentration affects of ATRA on nanoparticle characteristics.

| %ATRA | Size (nm) | PDI | Zeta Potential (mV) |
|---|---|---|---|
| 1% | 156.3 | 0.316 | -2.03 |
| 0.5% | 90.46 | 0.244 | -1.53 |
| 0.25 | 85.71 | 0.259 | -1.21 |

As might be expected, trends suggest that as ATRA concentration increases, size and PDI increase, while zeta potential decreases, suggesting that for zein-PEG, at least, there may be a predictable lessening of aggregation as ATRA concentration increases.

ATRA loading and encapsulation efficiency as a function of phase separated zein-casein and spray dried PEG zein is shown in Table 16 (analysis performed using HPLC).

Table 16. Efficiency of loading and encapsulation using ATRA and ZC and zein-PEG nanoparticles.

| Formulation | Loading efficiency % | Encapsulation efficiency % |
|---|---|---|
| ZC nanoparticles | 4.1 | 88.56 |
| Spray dried PEG-Zein | 1.17 | 61 |

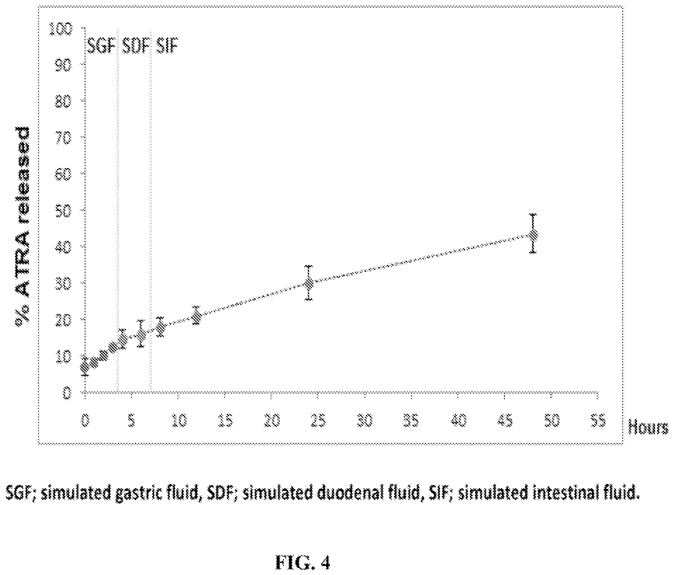

The results indicate that ATRA loaded zein-casein nanoparticles are stable in storage conditions and that there were no remarkable changes in the particle size or ATRA content for up to 60 days. Further, it seems that ZC nanoparticles exhibit greater loading and encapsulation efficiency than zein-PEG. In vitro release data for these formulations are shown in FIG. 4.

Example 7. ZC nanoparticles and saquinavir (SQ) combinations (not according to the invention).

Table 17 shows the characteristics of saquinavir (SQ) loaded zein casein nanoparticles prepared by phase separation.

Table 17. SQ containing nanoparticle characteristics prepared by phase separation.

|  | Size (nm) | PDI | Zeta Potential |
|---|---|---|---|
| ZC | 91.19±1.64 | 0.105±0.042 | -44.8±2.9 |
| SAQ loaded ZC | 116.1 | 0.188 | -39.5 |

Table 18 shows the SQ content and encapsulation efficiency of ZC nanoparticles by HPLC.

Table 18. SQ encapsulation and loading efficiencies.

|  | SQ-ZC nanoparticles |
|---|---|
| Loading efficiency % | 0.94±0.31 |
| Encapsulation Efficiency % | 98.2±4.6 |

Example 8. Materials and Methods for Zein-Lactoferrin Nanoparticles.

Phase separation method

0.2% (w/v) bovine lactoferrin (Fonterra Cooperative Group, Auckland, New Zealand) was dissolved in 0.1 M citrate buffer solution. 15 mg white zein from corn (Grade F6000) was dissolved in 2 ml of 90% EtOH. To this ethanolic solution, saquinavir was added from stock solution in concentration range from about 0.1 to 1 mg. The organic phase was added drop wise to 0.1 M citrate buffer solution containing lactoferrin under probe sonication. The probe sonication was set to 38% amplitude for 10 min (10 sec on and 1 sec off cycle) and sonicated over energy of 500 kJ. The zein-lactoferrin dispersion was left under magnetic stirrer (300 rpm) for 4 hours to evaporate the EtOH. Furthermore, the nanoparticles were separated using Millipore centrifugal filters (30k MWCO) and washed several times with deionized water using a pipette. Optionally, a cryoprotectant was added (trehalose) and then the mixture was placed in -80°C followed by lyophilization for 48 hours at -105°C/100 mTorr vacuum. Finally, the formulation was stored in closed vials in a dessicator until further use.

Genipin crosslinked Zein-lactoferrin (ZLF) nanoparticles.

ZLF nanoparticles were crosslinked by adding genipin (0.5-10 mg/ml) as a crosslinking agent to the organic phase (EtOH), followed by the steps as outlined above. Subsequently, the resulting crosslinked nanoparticles were purified, washed and lyophilized as outlined above.

Example 9. ZLF nanoparticles Physiocochemical Characterization

Characterization of particle size, distribution and zeta potential were determined by dynamic laser light scattering using the Malvern Zetasizer-S 3600 (Malvern Instruments Inc. Southborough, MA).
Results

|  | | Size (d.nm) | % Intensity | Width (d.nm) | |
|---|---|---|---|---|---|
| Z-Average (d.nm): 194.9 | Peak 1: | 221.1 | 100.0 | 74.69 | |
| Pd1: 0.122 | Peak 2: | 0.000 | 0.0 | 0.000 | |
| Intercept: 0.929 | Peak 3: | 0.000 | 0.0 | 0.000 | |
| Result Quality: Good | | | | | |

Table 19. Ratio affect on ZLF nanoparticle characteristics.

| Zein:LF ratio | Size (nm) | PDI | Zeta Potential (mV) |
|---|---|---|---|
| 1:1 | 301.4 | 0.306 | -6.15 |
| 1:2 | 141.3 | 0.244 | 34.3 |
| 1:4 | 174.7 | 0.275 | 38.6 |

Table 20. Source affects on ZLF nanoparticle characteristics.

| Source | Size (nm) | PDI | Zeta Potential (mV) |
|---|---|---|---|
| LF from Sigma | 293.7 | 0.319 | 13.1 |
| LF from Fronterra | 155.9±14.5 | 0.22±0.027 | 38.5±4.32 |
| Genipin cross-linked ZLF | 168.2±9.6 | 0.247±0.018 | 42.9±2.33 |

Table 21. Buffer affects on ZLF nanoparticle characteristics.

| Aqueous Phase | Size (nm) | PDI | Zeta Potential (mV) |
|---|---|---|---|
| Phosphate Buffer | 430.1 | 1 | -7.7 |
| Citrate Buffer | 155.9±14.5 | 0.22±0.027 | 38.5±4.32 |

Table 22. Characteristics of ATRA loaded-ALF nanoparticles.

| | Size (nm) | PDI | Zeta Potential (mV) |
|---|---|---|---|
| ATRA-ZLF | 167.75±8.7 | 0.277±0.014 | 31.3±2.97 |
| Saquinavir loaded ZLF nanoparticles | | | |

Table 23. Characteristics of SQ loaded-ALF nanoparticles.

| | Size (nm) | PDI | Zeta Potential (mV) |
|---|---|---|---|
| SQ-ZLF | 182.6±8.7 | 0.273±0.009 | 35.1±4.31 |

Example 10. Morphological Analysis

Atomic force microscopy studies

Approximately 1 mg of nanoparticles in 1 mL of distilled water was used for AFM analysis. 100 $\mu$l of the dispersion was placed on a freshly cleaved mica surface using polyethylene amine coated glass cover slip and air dried overnight. AFM images were collected in the scan area of 1 $\mu$m.

Transmission electron microscopy (TEM):

Images were acquired using a Tecnai Sprint G2 Twin (FEI Company) transmission electron microscope, Department of Chemistry Churchill-Haines Laboratories, University of South Dakota, Vermillion. The instrument was operated at an accelerating voltage of 120 kV. The nanoparticle specimen were prepared by dropping dilute dispersions of ZLF nanoparticles onto carbon-coated 200 mesh copper grids (Electron Microscopy Sciences) and allowed to evaporate overnight. All electron micrographs were recorded using a SC200 CCD camera coupled to the TEM. The image analysis was performed using DitialMicrograph software. TEM image shows that ZLF nanoparticles are spherical, around 100 nm in size with core-shell characteristics. The scale bar is 50 nm (not shown).

Table 24. Influence of pH on ZLF nanoparticle size and polydispersity index.

| pH | PS (nm) | PDI |
|---|---|---|
| 2 | 207.21± 12.24 | 0.194± 0.012 |
| 3 | 199.55± 17.93 | 0.189± 0.035 |
| 4 | 205.32± 18.43 | 0188± 0.018 |
| 5 | 201.63± 18,71 | 0.167± 0.018 |
| 6 | 195.91± 14.65 | 0.188± 0.005 |
| 7 | 199.75± 14.15 | 0.185± 0.035 |
| 8 | 201.35± 19.11 | 0.197± 0.021 |
| 9 | 201.15± 17.29 | 0.189± 0.013 |

Zeta potential fig. the zeta potential was highly positive (+30 mV) from pH 2 to 4, moderately positive (+20 mV) at pH 5 to 6. ALF nanoparticles were stable to aggregation across the entire pH range examined. While not being bound by theory, this may be attributable to the shell steric repulsion which is typical of linger range because of the hydrophilic carbohydrate groups attached to the polypeptide chain of lactoferrin and the higher molecular weight of LF compared to β-casein.

Table 25. Influence of ionic strength on ZLF nanoparticle size, distribution and zeta potential.

| Ionic Strength (mM; NaCl) | PS | PDI | Zeta Potential |
|---|---|---|---|
| 10 | 187.95± 24.4 | 0.188± 0.018 | 40.21± 2.838 |
| 20 | 174.35± 27.08 | 0.197± 0.016 | 26.85± 4.849 |
| 50 | 183.9± 29.6 | 0.184± 0.045 | 20.32± 3.788 |
| 100 | 175.35± 24.11 | 0.187± 0.021 | 14.85± 2.758 |
| 150 | 169.25± 14.21 | 0.183± 0.017 | 9.55± 3.869 |
| 200 | 175.1± 32.9 | 0.191± 0.030 | 6.19± 2.687 |

From Table 25 there was no change in the particle size due to changes in ionic strength. However, charge reversal was observed going from highly positive to slightly positive with increasing salt concentration. While not being bound by theory, this data suggests that there may have been some binding of anionic chloride ions to positively charged groups on the shell of the ZLF nanoparticles.

Reconstructed formulations in stimulated gastric fluids showed and array of solution characters. For example, zein-ethanolic dispersions showed milky aggregates, while zein-casein (ZC) nanoparticles show a slight turbidity, with a bluish gleam, and ZLF shows slight turbidity with a reddish gleam.

Example 11. Drug content and encapsulation efficiency.

ATRA or saquinavir content and encapsulation efficiency was measured by HPLC method. Briefly, 4 mg of drug loaded ZLF nanoparticles were dispersed in 1 ml of DI water, and then centrifuged at 1400 rpm for 10 min. The supernatant was discarded and the pellet was kept for analysis. Carefully, the pellet was dispersed in 50% EtOH/CB and probe sonicated for 5 min followed by bath sonication to ensure complete solubility. A standard cure was generated on the same day for ATRA or saquinavir quantification.

For Nile Red (Sigma, St. Louis, MO), loaded ZLF particles the fluorescence of Nile Red was measured in a spectrophotometer (e.g., SpectromaxM2, Molecular Devices, Sunnydale, CA) at excitation and emission wavelength of 559 and 629 nm.

Chromatographic conditions for ATRA were as follows:

Column: C-18 column, 100Å, 5 um, 4.6 mm x 150 mm; Mobile phase: acetonitrile (1.13 ml/min)-1% w/v ammonium acetate buffer (0.12 ml/min); Detection wavelength: 342 nm; Injection volume: 50 μl; Run time: 10 min.

Chromatographic conditions for saquinavir were as follows:

Column: C-18 column, 3.5 μm, 4.6 mm x 75 mm; Mobile phase: acetonitrile (0.48 ml/min)-0.7% w/v ammonium acetate buffer (0.32/min); Detection wavelength: 240 nm; Injection volume: 20 μl; Running time: 8 min.

Table 26. Characteristics for ZLF nanoparticles encapsulating Nile Red, ATRA and SQ.

| | PS | PDI | Zeta potential | Drug loading % | Encapsulation efficiency % |
|---|---|---|---|---|---|
| Nile Red | 175± 6.8 | 0.251± 0.02 | 28.6±3.12 | 0.281 | 82.5 |
| ATRA | 167.75± 8.7 | 0.277± 0.014 | 31.3± 2.97 | 4.1 | 88.56 |
| Saquinavir | 182.6± 10.2 | 0.237± 0.009 | 35.1± 4.31 | 1.7 | 85.2±8.2 |
| ATRA: all trans retinoic acid. | | | | | |

For as comparison of encapsulation and loading efficiency with zein-PEG, see Figs.).

Example 12. Differential Scanning Calorimetry (DSC).

The physical state of saquinavir in the formulation was determined by DSC using a DSC Q200 (TA Instruments, Inc., USA). Accurately weighed samples (5 mg) were crimped into aluminum crimp pans (T Zero Lid #T100819) and heated at the rate of 10°C/min from 23 to 300°C under a nitrogen atmosphere (flow rate 20 ml/min). A blank aluminum cell was used as a reference. The thermograms (not shown) were processed using TA Universal Analysis software (TA Instruments, Inc., USA).

Example 13. In vitro drug release studies.

Drug release kinetics was evaluated in simulated gastric and intestinal fluids by measuring the concentration of drug left in the nanoparticles (tube method) with concurrent changing of the release medium conditions. Briefly, 10 mg of drug loaded ZLF nanoparticles were suspended in 2 ml Eppendorf tubes containing the release medium with enzymes. The pH of the release medium was adjusted using an Accumet pH meter. "Fed" state simulated gastrointestinal fluid (FeSSGIF) and "Fasted" state simulated gastrointestinal fluid (FaSSGIF) were prepared from simulated intestinal fluid (SIF) powder (Biorelevant, Croydon, Surrey, UK), which contains sodium taurocholate and lecithin in a specific ratio. At different time points, two samples were taken and centrifuged at 14,000 rpm for 10 min. The supernatant was carefully removed using a 1 ml pipette and the remaining pellet was dissolved in 50% EtOH for HPLC analysis. The concentration of drug was determined using HPLC as previously described herein.

Example 14. In vitro drug release studies in common foods and beverages

For ATRA release in simulated GIT fluids and common foods and beverages, ATRA was quantified using $^3$H-ATRA. Concurrent sampling was carried out to simulate the fed and fasted state. Briefly, 10 mg of ATRA-loaded ZLF nanoparticles were suspended n 2 ml Eppendorf tubes containing release medium, apple juice or milk (3% fat). The tubes were placed in a shaker at 37°C, at 600 rpm. At different time points, samples were removed and centrifuged at 14,000 rpm for 10 min. The supernatant was carefully removed using a 1 ml pipette and the remaining pellet was dissolved in 50% EtOH. ATRA content was analyzed using radiochemical analysis (see FIG 4).

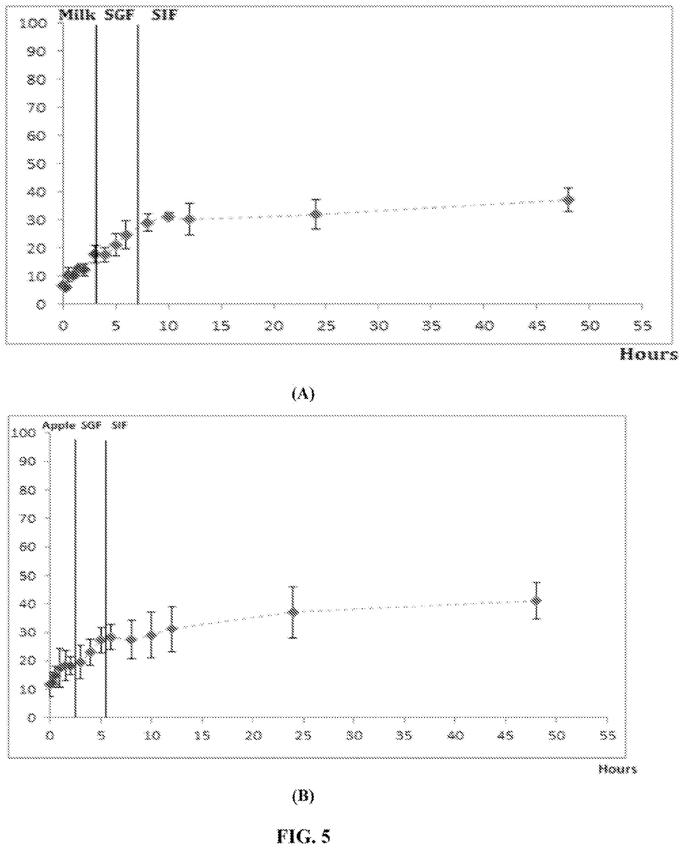

For saquinavir release in common foods and beverages, analysis was carried out by extraction and quantification using HPLC analysis as previously described. Briefly, the saquinavir loaded ZLF nanoparticles were centrifuged, and the resulting pellet was frozen at - 80°C and lyophilized. The lyophilized material was then extracted with 50% EtOH and bath sonicated. The resulting material was then extracted with 90% EtOH and bath sonicated. After sonication, the material was then centrifuged, and the resulting supernatant was injected into an HPLC (see FIG. 5).

Example 15. Spray drying method

A spray drying process was carried out by dissolving both zein and lactoferrin in binary ethanolic solution (i.e., 55% EtOH/citrate buffer[CB]) so that the pH was 4 to 5 and the total concentration of proteins was between 1 to 10% w/v. Following dissolution, the solution was bath sonicated and visually examined before spraying to ensure complete solubility. Saquinavir was added from ethanolic stock solution at a concentration of 1% and mixed prior to spray drying. The spray drying was performed using a Nano Spray Dryer (B-90, BÜCHI) at room temperature for 6-8 hours. The operating parameters were as follows

Spray drying through a 4 um spray mesh, spray rate 100%, the inlet temperature was set to 100°C and nitrogen flow rate of 150L/min.

Dry particles were collected from the collecting drum using a suitable scraper and stored in a dessicator until use.

Subsequently, ZLF nanoparticles may be crosslinked using genipin as a crosslinking agent. After spray drying, the ZLF nanoparticles were crosslinked by incubation the nanoparticle dispersion in a 10 mM citrate buffer solution containing genipin (1.0 mg/ml) for 3 to 4 hours at room temperature. The resulting nanoparticles were purified using Millipore centrifuge filters (30k MWCO) and washed with DI water. Optionally, cryoprotectant (e.g., trehalose) was added. Following that, the nanoparticles were placed at - 80°C followed by lyophilization for 48 hours at -105°C/100 mTorr vacuum. Finally, the lyophilized particles were stored in a dessicator until use.

Table 27. Physicochemical characteristics of spray dried ZLF nanoparticles.

| | PS | PDI | Zeta Potential | Drug loading % | Encapsulation efficiency % |
|---|---|---|---|---|---|
| ZLF | 141.5±5.6 | 0.205±0.17 | 38.6±2.58 | - | - |
| Genipin-ZLF | 185.61±11.7 | 0.254±0.12 | 35.9±3.44 | - | - |
| Saquinavir-ZLF | 202.6± 14.6 | 0.265±0.099 | 29.2±1.88 | 1 | 90.1 |

Example 16. In vitro evaluation of zein-lactoferrin nanoparticles in comparison to previously developed zein based nanoparticles.

Caco-2 cell culture

Caco-2 cells (ATCC, Manassas, VA) were kindly provided by Dr. Gunaje Jayarama (Department of Pharmaceutical Sciences, South Dakota State University, Brookings, SD) at passage #4. Cells were seeded at 1 x 10[6] cells onto TRANSWELL® polyester filter membranes (pore size 0.4 um, growth area 4.67 cm$^2$) in 6 welled sterile plates (TRANSWELL®, Corning Costar Corp., Cambridge, MA). The cells were grown in DMEM (4.5 g/L glucose) medium supplemented with 20% FBS, 1% non-essential amino acids, 1% L-glutamine, streptomycin (100 $\mu$g/ml) and penicillin (100 IU/ml). The cultures were maintained in an atmosphere of 5% $CO_2$ and 95% air, and 95% relative humidity at 37°C ($CO_2$ incubator, Galaxy 170S). The growth medium was changed every day in the first two weeks followed by three times a week until time of use. When reaching confluency, cells were trypsinized and passage numbers 25-35 were used in the experiments. The cells were allowed to differentiate in the TRANSWELL® filter membrane during 3 weeks in complete growth medium.

The permeability of saquinavir and saquinavir loaded ZLF nanoparticles were studied across Caco-2 cell monolayers in an absorptive direction apical to basolateral (AP-BL) or secretory direction basolateral to apical (BL-AP). Before the permeability experiments, the cell monolayers were rinsed twice with HBSS, pH 7.4, and equilibrated under experimental conditions for 20 min. Then, the apical fluid was replaced by 1.5 ml of experimental solution. Transepithelial electrical resistance (TEER) was measured using an EVOM meter (World Precision Instruments, Inc., Sarasota, FL) to ensure monolayer confluence and integrity. TEER was calculated as follows:

$$TEER(\Omega \bullet cm^2) = (R_{total} - R_{filter}) \times A$$

Where R($\Omega$) is the measured resistance and A is the surface area (cm$^2$). Cells were used for experimentation with a TEER value grater than or equal to 1000$\Omega \bullet$ cm$^2$. At time zero, samples (0.5 ml) from the apical chamber were withdrawn to determine the initial saquinavir concentration. Monolayers were incubated at 37°C under controlled atmosphere (5% $CO_2$, 95% relative humidity). During 3 hours, samples (1 ml) were withdrawn from basolateral compartment and immediately replaced by fresh buffer solution. All experiments were performed in triplicate. Results are expressed as apparent permeability coefficient (Papp), calculated according to:

$$Papp \ (cm/s) = (dQ/dt) \times (1/A.C_0)$$

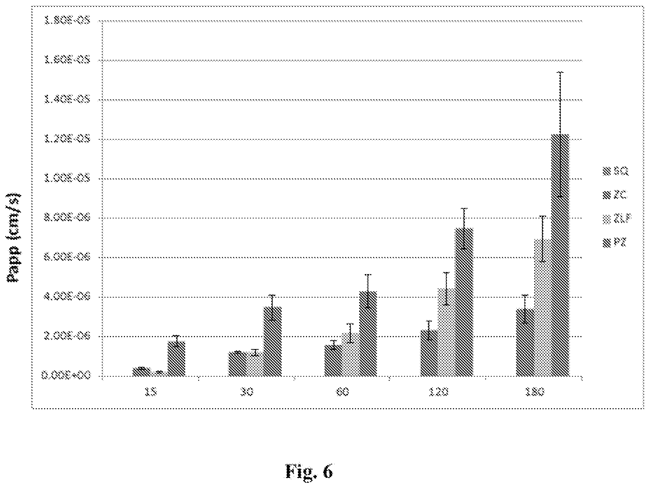

Where dQ/dt is the flux of saquinavir across Caco-2 monolayer, $C_0$ is the initial concentration of saquinavir in the apical chamber, and A is the surface area of the TREANSWELL® filter (4.71 cm$^2$).
Saquinavir content after extraction was analyzed in the samples using the HPLC as recited herein. (See FIG. 6)
Bidirectional transport and efflux ratio.
The ratio of effective permeability for saquinavir or efflux ratio was calculated as follows:

$$EfR = (Papp \ B\text{-}A/Papp \ A\text{-}B)$$

Where (Papp B-A) is the apparent permeability coefficient for saquinavir in the direction from the basolateral to the apical, and (Papp A-B) is the apparent permeability coefficient for saquinavir in the direction from the apical to the basolateral. (see FIG). The results can be seen in Table 28.

Table 28. Transport and efflux ratios for SQ.

| | $P_{app(A\text{-}B)}$ (cm/s $\times$ 10$^{-6}$) | $P_{app(B\text{-}A)}$ (cm/s $\times$ 10$^{-6}$) | Efflux ratio |
|---|---|---|---|
| Saquinavir (20 $\mu$M) | 0.85±0.21 | 14.23±5.3 | 16.7 |
| SQ loaded ZC nanoparticles | 2.53±1.1 | 3.86±1.6 | 3.318 |
| SQ loaded ZLF nanoparticles | 1.83±1.2 | 1.07±0.72 | 0.889 |
| SQ loaded PZ | 3.03±0.8 | 4.13±1.4 | 1.509 |
| Transepithelial permeability of Nile Red loaded nanoparticles across Caco-2. | | | |

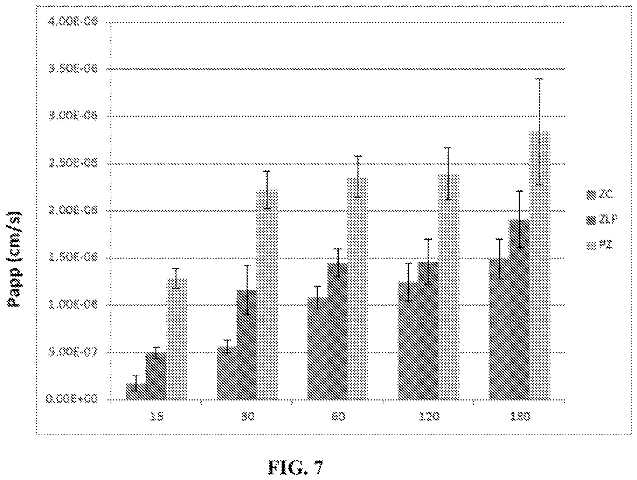

The permeability of NR and NR loaded ZLF nanoparticles were studied across Caco-2 cell monolayers as previously described. To quantify the NR content, the fluorescence was measured in a spectrophotometer (SpectromaxM2, Molecular Devices, Sunnydale CA) at extinction and emission wavelengths of 559 and 629 nm, respectively. (See, FIG. 7)

Example 17. Effect of nanoparticles on TEER.

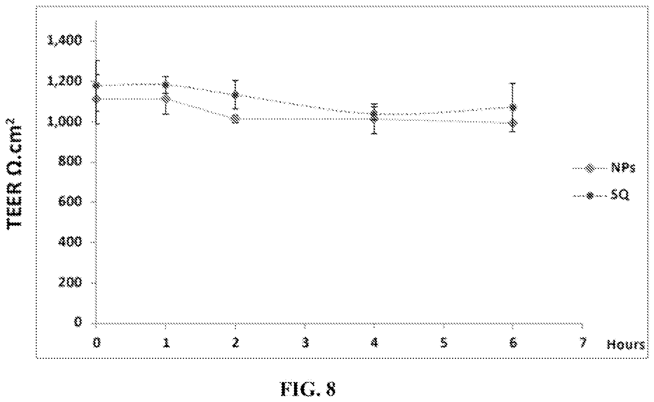

TEER was measured for each TRANSWELL® inserts. The electrode tip was balanced with HBSS buffer overnight in sterile condition before measurement. After nanoparticle treatment, TEER was measured at three different positions at room temperature. The final TEER (ohm) for each insert was the average of three reads during a 6 hour experiment. (See FIG. 8).

Example 18. Cellular uptake of Nile Red (NR) loaded nanoparticles

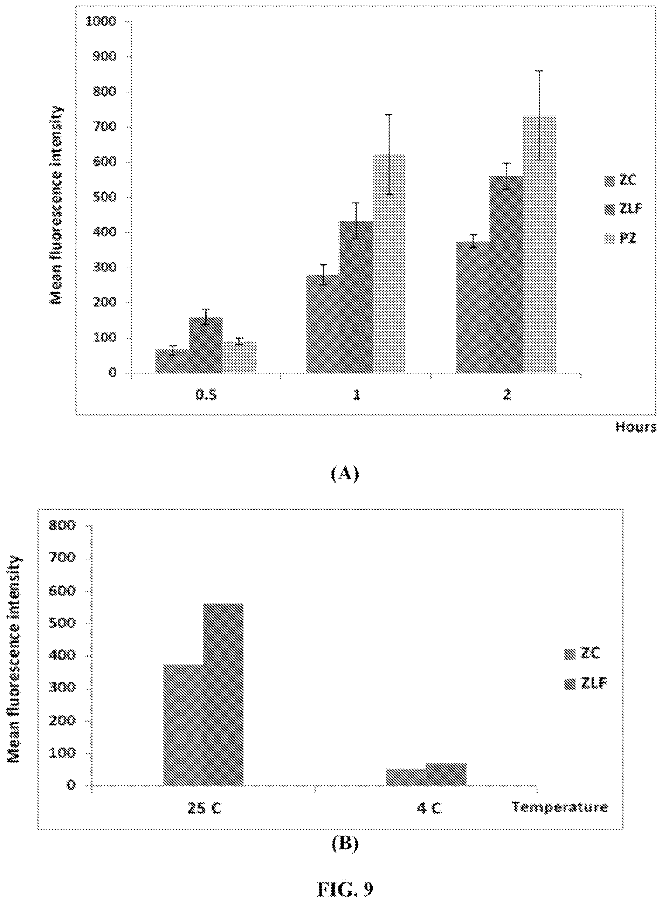

Cell uptake was performed using NR fluorescent probe (ex. 559 and em. 629 nm), encapsulated in Zein based nanoparticles. Caco-2 cells were seeded in a 6 well plate at a concentration of $2 \times 10^5$ cells/well and were allowed to adhere overnight. The next day, the culture medium was discarded and the cells were equilibrated at least for 30 min with HBSS before the uptake study. The cells were then treated with the nanoparticles at a concentration of 2 mg/ml for different time points (e.g., 30 min, 1 hour, and 2 hours) at 37°C. At the end of the incubation, the cells were washed with HBSS three times, detached from the wells by trypsinization, was with cold HBSS and fixed with 2% formaldehyde. The cells were kept at 4°C and analyzed using flow cytometry (FACS, BD Biosciences, San Jose, CA), the mean fluorescence value for each sample was obtained by compiling the fluorescence of 20,000 events. (see FIG. 9)

Example 19. Competition inhibition assay (Flow cytometry analysis)

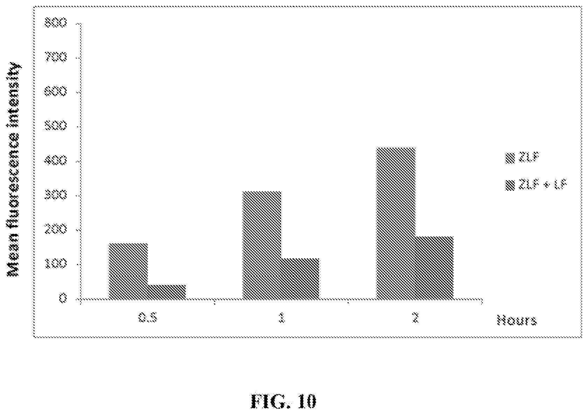

To verify that the uptake is mediated via lactoferrin receptor, a competitive experiment was performed in the presence of bovine lactoferrin. Cells were treated with 2mg/ml lactoferrin for 60 min prior to incubation with NR-ZLF nanoparticles (see FIG. 10).

Example 20. P-gp inhibition (Calcein AM assay)

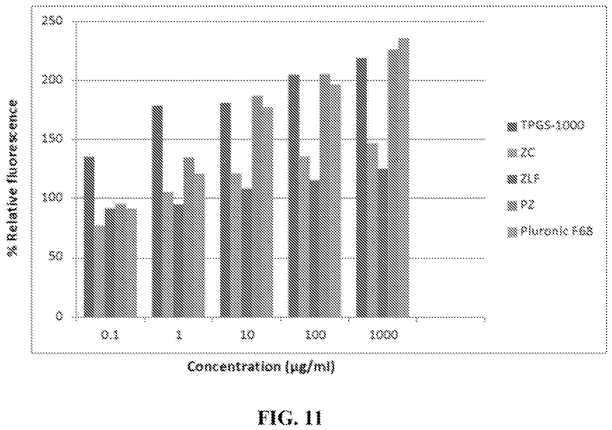

Caco-2 cells were seeded in black 96-well plates overnight. The next day, the cells were treated with 50 $\mu$l of blank nanoparticles diluted in HBSS buffer for 15, 30, and 60 min. Cells were washed three times and 50 $\mu$l of 0.25 $\mu$M calcein AM (Molecular Probes) was added to each well. The fluorescence of calcein was measured using a microplate reader with 485/589 excitation/emission at room temperature. To determine the P-gp inhibition the % relative fluorescence was calculated = (fluorescence after treatment - background fluorescence)/background fluorescence $\times$ 100. (See FIG. 11)

Visualization of ZO-1 distribution by confocal laser scanning microscopy (CLSM) or CLSM, cells were treated with nanoparticles at a concentration of 2 mg/ml for 30 min. The nanoparticles were removed by washing the cells three times with PBS. The cells were fixed with 0.25 ml of 4% paraformaldehyde for 20 min at room temperature. The cells were then permeablized using 0.25 Triton-X 100 in blocking solution, made of 1% (w/v) bovine serum albumin (BSA) for one hour. The cells were then washed and incubated with (1:1000) ZO-1 antibody (InVitrogen, Camarillo, CA) overnight at 4°C. The ZO-1 antibody binds to tight junctions (or zonula occludens). After removal of the ZO-1 antibodies, the cells were treated with 1% BSA and then incubated with 100 $\mu$l of alexa fluor 488 goat anti-mouse IgG for 1 hour at room temperature. The cells were washed with PBS and dried with DAPI overnight at 4°C. The data (images not shown) demonstrate that the NR loaded ZLF nanoparticles were able to cross the cell membranes.

In vitro release profile shows sustained release with not burst release particular to ZC nanoparticles. In vitro studies in Caco-2 cells demonstrated the ability of ZLF nanoparticles to increase the solubility and permeability of saquinavir. While not being bound by theory, this efficient cellular uptake is likely due to utilization of receptor mediated endocytosis pathways. The low efflux ratio for all nanoparticles compared to free saquinavir indicates escaping P-gp mediated efflux.

In light of these data, core-shell protein based nanoparticles represent an attractive approach to protect payloads from the external environment in of the GIT. The surrounding shell provides an additional diffusion layer that may serve as an extra barrier and that may prolong the release time with respect to a single protein. Thus, nanoparticles with core-shell characteristics may be pre-administered with foods without a significant loss of encapsulated drug, which is a common way for pediatric drug administration. Zein nanoparticles hold promise in terms of accessibility in developing countries and potential to improve patient compliance as well as oral bioavailability in a more stable, safe, scalable, and re-dispersible solid dosage. The next objective is to evaluate the in-vivo pharmacokinetics of these nanoparticles after oral administration.

Example 21. Animal study

Saquinavir (SQ) loaded protein based nanoparticles were prepared by the phase separation method as described herein. The oral pharmacokinetics of SQ was assessed in Sprague Dawley rate (6-8 weeks of age) weighing 250 g. Rats with catheterized jugular veins for blood sampling were purchased from Charles River (Wilmington, MA). All procedures were approved by the Institutional Animal Care and Use Committee (IACUC) at South Dakota State University. Rats were divided into 5 groups with 4 animals in each group as shown in Table 29.

Table 29. Animal groups and descriptions.

| Group | Dosing Method | Description |
|---|---|---|
| 1 | i.v. | SQ base |
| 2 | Oral | SQ suspended in 0.5% w/v sodium carboxymethyl cellulose |
| 3 | Oral | SQ loaded ZC nanoparticles (equivalent to 20 mg/Kg SQ base) |
| 4 | Oral | SQ loaded ZLF nanoparticles (equivalent to 20 mg/Kg SQ base) |
| 5 | Oral | SQ loaded PZ nanoparticles (equivalent to 20 mg/Kg SQ base) |
| ZC, zein-casein nanoparticles; ZLF, zein-lactoferrin nanoparticles; PZ, polyethylene glycol-zein nanopartilces; SQ, saquinavir. | | |

Rats were maintained on a 12 hr light/dark cycle at $24\pm2°C$ and provided with a standard rodent diet. Before starting the experiments, rats were fasted for 12 hours by had free access to water. SQ formulation was administered by oral gavage using a stomach tube. Blood samples (200 $\mu$l) were withdrawn from the jugular vein catheter cannula at different time points (0, 0.25, 0.5, 1, 2, 4, 6, 8, 12, 24, and 48 hrs) into (1 ml) heparinized blood collection tubes. The patency of the catheter was maintained by flushing it with sterile saline after each sampling. The samples were centrifuged for 15 min at 12,000 rpm then plasma was collected and stored at -80°C until analyzed by HPLC.

Sample preparation and Chromatography. Aliquots (50 $\mu$l) of sample plasma was transferred into a 1.5 ml centrifuge tube and 100 $\mu$l of borate buffer (pH 10) was added. Subsequently, 200 $\mu$l of a mixture of ethyl acetate-hexane (50:50) was added to extract the drug. The extraction was repeated twice and the organic phases were collected and evaporated to dryness under a gentle stream of nitrogen gas. The residue obtained was reconstituted with 50% EtOH and vortexed for 10 min. The analysis of SQ was made on a symmetry C-18 column (Waters Corporation, Milford, USA), 5 um, 250 x 4.6 mm i.d., at 25°C. The mobile phase was a mixture of 10 mM ammonium acetate buffer-acetonitrile (35:65 v/v) pumped at a flow rate of 1.0 ml/min. Detection was performed at a wavelength of 240 nm and retention time of 3.6 min. A limit of qualification of 10 ng/ml was obtained under the analytical method as described.

Non-compartmental analysis of SQ plasma concentrations was performed using PK Solutions 2.0 software (Summit Research Services, Montrose, CO). The following table (Table 30) shows pharmacokinetic parameters for AQ upon dingle oral administration of different protein based formulations in rats using a non-compartmental approach.

Table 30. Pharmacokinetic parameters for SQ analysis in vivo.

| Parameters | SQ Formulations | | | |
|---|---|---|---|---|
| | CMC suspension | ZC NPs | ZLF NPs | PZ NPs |
| Dose (mg/kg) | 20 | 20 | 20 | 20 |
| $T_{max}$ (h) | 2.0 | 1.5 | 4.0 | 4.0 |
| $T_{1/2}$ (h) | 0.2 | 2.5 | 1.8 | 1.2 |
| $C_{max}$($\mu$g/ml) | 135.117 | 60.690 | 86.015 | 46.537 |
| $K_e$ ($h^{-1}$] | 0.005 | 0.011 | 0.008 | 0.015 |
| $AUC_{\infty}$ (ughml$^{-1}$) | 8.2 | 191.2 | 173.2 | 67.8 |
| MRT (h) | 170.2 | 86.9 | 122.8 | 66.1 |
| $F_{abs}$ (%) | 1.42 | 33.04 | 29.96 | 11.73 |

(continued)

| Parameters | SQ Formulations | | | |
|---|---|---|---|---|
| | CMC suspension | ZC NPs | ZLF NPs | PZ NPs |
| $F_{rel}$ (%) | - | 2331.7 | 2112.2 | 826.8 |

ZC, zein-casein nanoparticles; ZLF, zein-lactoferrin nanoparticles; PZ, polyethylene glycol-zein nanoparticles; CMC, carboxymethyl cellulose; $C_{max}$, the maximum observed concentration; $T_{max}$, the time at maximum observed concentration; $t_{1/2}$, the time for concentration to be reduced by one-half in the elimination phase; $AUC_{\infty}$, the total area under the curve calculated using observed data points combined with an extrapolated value; $K_e$, elimination rate constant; MRT, first-order moment mean resistance time; $F_{abs}$, absolute bioavailability; $F_{rel}$, relative bioavailability.

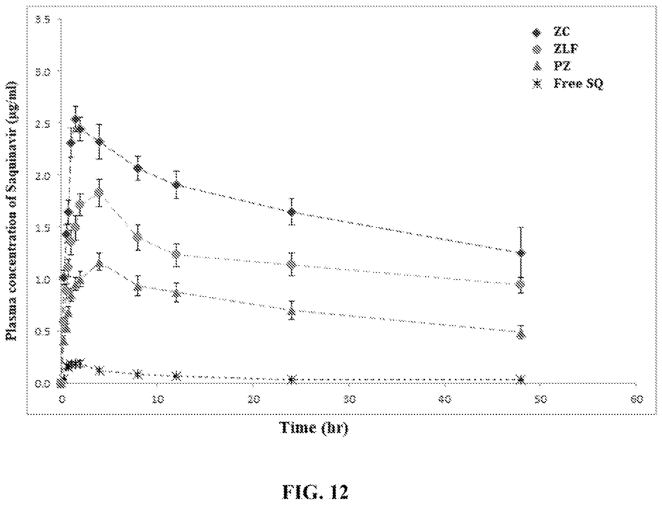

The plasma concentration profile for SQ in rats after oral administration may be seen in FIG 12. The bioavailability of SQ was enhanced from zein based nanoparticles. As can be seen from the profile, the drug is slowly released and the plasma concentration was maintained until about 48 hours in the blood stream.

**Claims**

1. A nanoparticle comprising at least two proteins, wherein a first protein is a prolamine and a second protein is lactoferrin, and wherein said nanoparticle exhibits a core-shell structure.

2. The nanoparticle of claim 1, wherein the prolamine protein comprises white zein, yellow zein, gliadin, hordein, or kafirin.

3. The nanoparticle of claim 2, further comprising a cargo molecule.

4. The nanoparticle of claim 3, wherein the cargo molecule is selected from the group BCS class II and class IV drugs.

5. The nanoparticle of claim 3, wherein the cargo molecule is a retinoid selected from the group consisting of retinol, 13-*trans*-retinoic acid (tretinoin) or all trans retinoic acid (ATRA), 13-*cis*-retinoic acid (isotretinoin), 9-*cis*-retinoic acid (alitretinoin), retinaldehyde, etretnate, acitretin, $\alpha$-carotene, $\beta$-carotene, $\gamma$-carotene, $\beta$-cryptozanthin, lutein, zeaxanthin, and combinations thereof.

6. The nanoparticle of claim 3, wherein the cargo molecule is saquinavir.

7. The nanoparticle of claim 1, wherein the nanoparticle is formed by spray drying or phase separation.

8. The nanoparticle of claim 1, further comprising cargo, wherein said cargo is a cell, protein, nucleic acid, antibody, growth factor, or a combination thereof.

9. The nanoparticle of claim 8, wherein said cargo is adsorbed to the surface of the nanoparticle.

10. The nanoparticle of claim 1, wherein the nanoparticle is cross-linked, and wherein a crosslinking agent is genipin.

11. The nanoparticle of claim 1, wherein the prolamine protein of the nanoparticle is PEGylated, wherein the PEG has a molecular weight of between 3 kDa and 20 kDa.

12. The nanoparticle of claim 1, wherein the nanoparticle is in the form of a dry, free flowing, colorless or white, non-hygroscopic powder.

13. The nanoparticle of claim 1, further comprising a diluent, an excipient, or carrier to form a pharmaceutically acceptable composition.

14. The nanoparticle of claim 13, wherein the composition is an oral formulation and is optionally contained in a food or a beverage.

**15.** A kit comprising:

a) a lyophilized powder or dispersion containing the nanoparticles of claim 1;
b) one or more buffers;
c) one or more labels;
d) one or more containers; and
e) an instruction manual, wherein the instruction manual discloses how to use the lyophilized powder.

## Patentansprüche

**1.** Nanopartikel, umfassend mindestens zwei Proteine, wobei ein erstes Protein ein Prolamin ist und ein zweites Protein Lactoferrin ist und wobei das Nanopartikel eine Kern-Schale-Struktur aufweist.

**2.** Nanopartikel nach Anspruch 1, wobei das Prolamin-Protein weißes Zein, gelbes Zein, Gliadin, Hordein oder Kafirin umfasst.

**3.** Nanopartikel nach Anspruch 2, des Weiteren umfassend ein Frachtmolekül.

**4.** Nanopartikel nach Anspruch 3, wobei das Frachtmolekül aus Arzneistoffen der BCS-Klasse II und der BCS-Klasse IV ausgewählt ist.

**5.** Nanopartikel nach Anspruch 3, wobei das Frachtmolekül ein aus der Gruppe bestehend aus Retinol, 13-*trans*-Retinsäure (Tretinoin) oder a11-*trans*-Retinsäure (ATRA), 13-*cis*-Retinsäure (Isotretinoin), 9-*cis*-Retinsäure (Alitretinoin), Retinaldehyd, Etretinat, Acitretin, α-Carotin, β-Carotin, γ-Carotin, β-Cryptoxanthin, Lutein, Zeaxanthin und Kombinationen derselben ausgewähltes Retinoid ist.

**6.** Nanopartikel nach Anspruch 3, wobei das Frachtmolekül Saquinavir ist.

**7.** Nanopartikel nach Anspruch 1, wobei das Nanopartikel durch Sprühtrocknung oder Phasentrennung gebildet ist.

**8.** Nanopartikel nach Anspruch 1, des Weiteren umfassend eine Fracht, wobei die Fracht eine Zelle, ein Protein, eine Nukleinsäure, ein Antikörper, ein Wachstumsfaktor oder eine Kombination derselben ist.

**9.** Nanopartikel nach Anspruch 8, wobei die Fracht an der Oberfläche des Nanopartikels adsorbiert ist.

**10.** Nanopartikel nach Anspruch 1, wobei das Nanopartikel vernetzt ist und wobei ein Vernetzer Genipin ist.

**11.** Nanopartikel nach Anspruch 1, wobei das Prolamin-Protein des Nanopartikels PEGyliert ist, wobei das PEG ein Molekulargewicht zwischen 3 kDa und 20 kDa aufweist.

**12.** Nanopartikel nach Anspruch 1, wobei das Nanopartikel in Form von trockenem, rieselfähigem, farblosem oder weißem, nicht hygroskopischem Pulver vorliegt.

**13.** Nanopartikel nach Anspruch 1, des Weiteren umfassend einen Verdünner, einen Hilfsstoff oder einen Trägerstoff zur Ausbildung einer pharmazeutisch verträglichen Zusammensetzung.

**14.** Nanopartikel nach Anspruch 13, wobei die Zusammensetzung eine orale Formulierung ist und wahlweise in einem Nahrungsmittel oder einem Getränk enthalten ist.

**15.** Kit, umfassend:

a) ein lyophilisiertes Pulver oder eine lyophilisierte Dispersion, das bzw. die die Nanopartikel nach Anspruch 1 enthält;
b) einen oder mehrere Puffer;
c) eine oder mehrere Etiketten;
d) einen oder mehrere Behälter; und
c) eine Gebrauchsanweisung, wobei die Gebrauchsanweisung Auskunft darüber gibt, wie das lyophilisierte Pulver zu verwenden ist.

**Revendications**

1. Nanoparticule comprenant au moins deux protéines, dans laquelle une première protéine est une prolamine et une deuxième protéine est la lactoferrine, et dans laquelle ladite nanoparticule présente une structure noyau-enveloppe.

2. Nanoparticule selon la revendication 1, dans laquelle la protéine de prolamine comprend de la zéine blanche, de la zéine jaune, de la gliadine, de l'hordéine ou de la kafirine.

3. Nanoparticule selon la revendication 2, comprenant en outre une molécule cargo.

4. Nanoparticule selon la revendication 3, dans laquelle la molécule cargo est sélectionnée dans le groupe de médicaments de la classe II et de la classe IV BCS.

5. Nanoparticule selon la revendication 3, dans laquelle la molécule cargo est un rétinoïde sélectionné dans le groupe composé par le rétinol, l'acide 13-*trans* rétinoïque (la trétinoïne) ou l'acide tout-*trans* rétinoïque (ATRA), l'acide 13-*cis* rétinoïque (l'isotrétinoïne), l'acide 9-*cis* rétinoïque (l'alitrétinoïne), le rétinaldéhyde, l'étrétinate, l'acitrétine, l'a-carotène, le β-carotène, le γ-carotène, la β-cryptoxanthine, la lutéine, la zéaxanthine et des combinaisons de ceux-ci.

6. Nanoparticule selon la revendication 3, dans laquelle la molécule de cargo est le saquinavir.

7. Nanoparticule selon la revendication 1, dans laquelle la nanoparticule est formée par la déshydratation par atomisation ou par la séparation de phases.

8. Nanoparticule selon la revendication 1, comprenant en outre une cargaison, dans laquelle ladite cargaison est une cellule, une protéine, un acide nucléique, un anticorps, un facteur de croissance ou une combinaison de ceux-ci.

9. Nanoparticule selon la revendication 8, dans laquelle la cargaison est adsorbée à la surface de la nanoparticule.

10. Nanoparticule selon la revendication 1, dans laquelle la nanoparticule est réticulée et dans laquelle un agent réticulant est la génipine.

11. Nanoparticule selon la revendication 1, dans laquelle la protéine de prolamine de la nanoparticule est PEGylée, dans laquelle le PEG à un poids moléculaire d'entre 3 kDa et 20 kDa.

12. Nanoparticule selon la revendication 1, dans laquelle la nanoparticule est sous la forme d'une poudre sèche, à écoulement libre, incolore ou blanche et non hygroscopique.

13. Nanoparticule selon la revendication 1, comprenant en outre un diluant, un excipient ou un support afin de former une composition pharmaceutiquement acceptable.

14. Nanoparticule selon la revendication 1, dans laquelle la composition est une formulation orale et est contenue facultativement dans un aliment ou dans une boisson.

15. Kit, comprenant :

a) une poudre ou une dispersion lyophilisée contenant les nanoparticules selon la revendication 1 ;
b) un ou plusieurs buffers ;
c) une ou plusieurs étiquettes ;
d) un ou plusieurs récipients ; et
e) un manuel d'instructions, dans lequel le manuel d'instructions explique comment utiliser la poudre lyophilisée.

Zein (15mg), in 90% ethanol (2.2 ml)

+ saquinavir (1 mg).

→ Vortex for 5 minutes and drop wise to the buffer solution

lactoferrin (0.2% w/v) in 0.1M Citrate buffer (20 ml)

→ Probe sonication for 10 minutes at 10°C with 38% amplitude; 10 seconds on and 1 second off cycle

Precipitate due to the phase change

→ Evaporate alcohol using magnetic stirrer (300-400rpm) for about 3-6 hours

Separate nanoparticles using centrifugal filters (Mol. Wt. 30k Daltons) at 40000rpm for 60 minutes

Wash the nanoparticles 2 times with D.I water.

Trehalose (15 mg) was added to nanoparticle dispersion was frozen until it forms a solid cake

Lyophilize (about 24 hours) at -100°C and 100 mTorr vaccum

Store in desiccator under refrigerated condition

**FIG. 1**

Zein (15mg), in 90% ethanol (2.2 ml) containing Geinipin (2mg) as a crosslinking agent.

+ saquinavir (1 mg).

→ Vortex for 5 minutes and drop wise to the buffer solution

lactoferrin (0.2% w/v) in 0.1M Citrate buffer (20 ml)

→ Probe sonication for 10 minutes at 10°C with 38% amplitude; 10 seconds on and 1 second off cycle

Precipitate due to the phase change

→ Evaporate alcohol using magnetic stirrer (300-400rpm) for about 3-6 hours

Separate nanoparticles using centrifugal filters (Mol. Wt. 30k Daltons) at 40000rpm for 60 minutes

Wash the nanoparticles 2 times with 0.1 water

Trehalose (15 mg) was added to nanoparticle dispersion was frozen until it forms a solid cake

Lyophilize (about 24 hours) at -100°C and 100 mTorr vaccum

Store in desiccator under refrigerated condition at 2-8°C

FIG. 2

White Zein (15mg), ATRA (1mg) in 90% ethanol (2 ml)

β-Casein (30 mg) in 10mM CB pH 7.0 (20 ml)

Vortex until it completely dissolved and add drop wise

In 55% (Ethanol/CB) (40 ml)

Bath sonication for 10 minute

Keeps in ice bath

Spray dry using BÜCHI B-90 through a 4 μm spray mesh in closed mode

Inlet temp. 115 °C, Spray rate 100% and nitrogen flow rate of 90 L/min

Collect the particles using a suitable scraper Store in desiccator under refrigerated condition

FIG. 3

SGF; simulated gastric fluid, SDF; simulated duodenal fluid, SIF; simulated intestinal fluid.

**FIG. 4**

(A)

(B)

FIG. 5

**Fig. 6**

**FIG. 7**

**FIG. 8**

**(A)**

**(B)**

**FIG. 9**

**FIG. 10**

**FIG. 11**

**FIG. 12**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5679377 A
- US 2012195947 A1
- WO 2009137112 A, Perumal
- US 5254673 A, Cook
- US 5851538 A, Froix
- US 4938949 A, Borch

**Non-patent literature cited in the description**

- **BERNSTEIN et al.** *Biomaterials,* 2005, vol. 26, 109-115
- **LOPEZ ; MURDAN.** *J Microencapsulation,* 2006, vol. 23, 303-314
- **ZHONG et al.** *Food Biophysics,* 2008, vol. 3, 186-190
- **PARRIS et al.** *J Agric Food Chemistry,* 2005, vol. 53, 4788-4792
- **ASHOK R. PATEL et al.** Sodium Caseinate Stabilized Zein Colloidal Particles. *Journal of Agricultural and Food Chemistry,* 08 December 2010, vol. 58 (23), ISSN 0021-8561, 12497-12503
- **HUAIQIONG CHEN et al.** Processes improving the dispersibility of spray-dried zein nanoparticles using sodium caseinate. *Food Hydrocolloids,* 01 March 2014, vol. 35, ISSN 0268-005X, 358-366
- **R.J. LEWIS.** Hawley's Condensed Chemical Dictionary. John Wiley & Sons, 2001
- **SHUKLA.** Zein: the industrial protein from corn. *Ind Crops Prod,* 2001, vol. 13, 171-92
- *Fed Register,* 1985, vol. 50, 8997-8999
- **LAWTON.** *Cereal Chem,* 2002, vol. 79 (1), 1-18
- **ORFANOS et al.** *Drug,* 1997, vol. 53, 358-388
- **MELO et al.** *J Control Release,* 2009, vol. 138, 32-39